# EUROPÄISCHE PATENTSCHRIFT

(11) **EP 1 176 987 B1**
(45) Veröffentlichungstag und Bekanntmachung des Hinweises auf die Patenterteilung: **11.02.2004**
(21) Anmeldenummer: 00922560.8
(22) Anmeldetag: 28.03.2000
(51) Int. Cl.: A61K 49/00, C09B 23/02, C09B 23/00, C09B 23/08

(54) **KURZKETTIGE PEPTID-FARBSTOFFKONJUGATE ALS KONTRASTMITTEL FÜR DIE OPTISCHE DIAGNOSTIK**
SHORT-CHAIN PEPTIDE DYE CONJUGATES USED AS CONTRAST AGENTS FOR OPTICAL DIAGNOSTICS
CONJUGUES DE COLORANTS AYANT DES PEPTIDES A COURTE CHAINE UTILISES COMME AGENTS DE CONTRASTE POUR LE DIAGNOSTIC OPTIQUE

(30) Priorität: 09.04.1999 DE 19917713
(43) Veröffentlichungstag der Anmeldung: 06.02.2002
(60) Teilanmeldung: 02090268.0
(73) Patentinhaber: Institut für Diagnostikforschung GmbH an der Freien Universität Berlin, 13353 Berlin (DE)
(72) Erfinder: LICHA, Kai, D-14612 Falkensee (DE); BECKER, Andreas, 85570 Markt Schwaben (DE); SEMMLER, Wolfhard, D-69120 Heidelberg (DE); WIEDENMANN, Bertram, D-12205 Berlin (DE); HESSENIUS, Carsten, D-12049 Berlin (DE); VOLKMER-ENGERT, Rudolf, D-13349 Berlin (DE); SCHNEIDER-MERGENER, Jens, D-10717 Berlin (DE); BHARGAVA, Sarah, D-12049 Berlin (DE)
(74) Vertreter: Seuss, Thomas
(86) Internationale Anmeldenummer: PCT/EP2000/002697
(87) Internationale Veröffentlichungsnummer: WO 2000/061194

(56) Entgegenhaltungen:
- EP-A- 0 591 820
- WO-A-90/06949
- WO-A-96/30055
- WO-A-97/40104
- WO-A-98/47538
- WO-A-98/57667
- DE-A- 19 649 971
- DE-A- 19 817 517
- JP-A- 1 088 537
- JP-A- 1 091 134
- US-A- 5 382 654
- US-A- 5 627 027
- US-A- 5 753 205
- US-A- 5 824 772
- US-A- 5 849 261
- REUBI J -C: "THE ROLE OF PEPTIDES AND THEIR RECEPTORS AS TUMOR MARKERS" ENDOCRINOLOGY AND METABOLISM CLINICS OF NORTH AMERICA,US,W.B. SAUNDERS COMPANY, PHILADELPHIA, Bd. 22, Nr. 4, 1. Dezember 1993 (1993-12-01), Seiten 917-939, XP000578548 ISSN: 0889-8529
- DATABASE WPI Section Ch, Week 199249 Derwent Publications Ltd., London, GB; Class B04, AN 1992-401811 XP002156179 & JP 04 297498 A (SEIKAGAKU KOGYO CO LTD), 21. Oktober 1992 (1992-10-21)
- LICHA K ET AL: "Highly parallel nano-synthesis of cleavable peptide-dye conjugates on cellulose membranes" TETRAHEDRON LETTERS,ELSEVIER SCIENCE PUBLISHERS, AMSTERDAM,NL, Bd. 41, Nr. 11, März 2000 (2000-03), Seiten 1711-1715, XP004202255 ISSN: 0040-4039
- RIEFKE ET AL: "In vivo characterization of cyanine dyes as contrast agents for near-infrared imaging" PROCEEDINGS OF THE SPIE,US,SPIE, BELLINGHAM, VA, Bd. 2927, 9. September 1996 (1996-09-09), Seiten 199-208, XP002037276
- LICHA ET AL: "Synthesis and characterization of cyanine dyes as contrast agents for near-infrared imaging" PROCEEDINGS OF THE SPIE,US,SPIE, BELLINGHAM, VA, Bd. 2927, 9. September 1996 (1996-09-09), Seiten 192-198, XP002079648
- LICHA ET AL: "Synthesis and characterization of cyanine dye - poly(ethylene glycol) conjugates as contrast agents for in vivo fluorescence imaging" PROCEEDINGS OF THE SPIE,US,SPIE, BELLINGHAM, VA, Bd. 3196, 6. September 1997 (1997-09-06), Seiten 98-102, XP002079471
- MANK A J G ET AL: "VISIBLE DIODE LASER-INDUCED FLUORESCENCE DETECTION IN LIQUID CHROMATOGRAPHY AFTER PRECOLUMN DERIVATIZATION OF AMINES" ANALYTICAL CHEMISTRY,US,AMERICAN CHEMICAL SOCIETY. COLUMBUS, Bd. 67, 1995, Seiten 1742-1748, XP002012845 ISSN: 0003-2700
- DELIGEORGIEV T G ET AL: "Preparation of Monomethine Cyanine Dyes for Nucleic Acid Detection" DYES AND PIGMENTS,GB,ELSEVIER APPLIED SCIENCE PUBLISHERS. BARKING, Bd. 37, Nr. 3, 1. Mai 1998 (1998-05-01), Seiten 205-211, XP004115887 ISSN: 0143-7208
- DATABASE WPI Section Ch, Week 199434 Derwent Publications Ltd., London, GB; Class E23, AN 1994-273908 XP002166231 & JP 06 202260 A (KONICA CORP), 22. Juli 1994 (1994-07-22)
- JAEHDE ULRICH ET AL: "Quantification and visualization of the transport of octreotide, a somatostatin analogue, across monolayers of cerebrovascular endothelial cells." PHARMACEUTICAL RESEARCH (NEW YORK), Bd. 11, Nr. 3, 1994, Seiten 442-448, XP000992868 ISSN: 0724-8741
- SONG Z -M ET AL: "Characterization of myenteric interneurons with somatostatin immunoreactivity in the guinea-pig small intestine." NEUROSCIENCE, Bd. 80, Nr. 3, 1997, Seiten 907-923, XP000992866 ISSN: 0306-4522
- BECKER, ANDREAS ET AL: "Novel receptor-targeted contrast agents for optical imaging of tumors" PROC. SPIE-INT. SOC. OPT. ENG., VOL. 3924, NO. MOLECULAR IMAGING: REPORTERS, DYES, MARKERS, AND INSTRUMENTATION, PAGE(S) 41-47, 23. - 24. Januar 2000, XP000992960
- BECKER, ANDREAS ET AL: "Cyanine dye labeled vasoactive intestinal peptide and somatostatin analog for optical detection of gastroenteropancreatic tumors" ANN. N. Y. ACAD. SCI., 2000, VOL. 921, NO. VIP, PACAP, GLUCAGON, AND RELATED PEPTIDES, PAGE(S) 275-278, XP000992937
- KOZICZ, TAMAS ET AL: "Immunohistochemical evidence for PACAP and VIP interaction with Met-Enkephalin and CRF containing neurons in the bed nucleus of the stria terminalis" ANN. N. Y. ACAD. SCI., VOL. 865, NO. VIP, PACAP, AND RELATED PEPTIDES, PAGE(S) 523-528, Januar 1999 (1999-01), XP000971509

## Beschreibung

Die Erfindung betrifft Verbindungen zur Tumordiagnostik, bestehend aus Konjugaten von Farbstoffen mit kurzkettigen Peptiden, die vom vasoaktiven intestinalen Peptid, vom Somatostatin oder vom Neurotensin abgeleitet sind, die Verwendung dieser Verbindungen als optische Diagnostika, und diese Verbindungen enthaltende diagnostische Mittel.

Krankheitsbedingte Veränderungen spiegeln sich auf zellulärer Ebene oft in einer gegenüber dem Normalzustand veränderten Rezeptorverteilung oder -expression wieder. Diese Unterschiede können sowohl quantitativer Art (z. B. Menge der Transferrin-Rezeptoren auf proliferierenden Zellen) oder aber auch qualitativer Art sein (z. B. Expression von Vascular endothelial growth factors, VEGF). Bisher werden Ansätze zur Abbildung einer pathologischen Rezeptorexpression oder -verteilung wegen der notwendigen Sensitivität des Detektionsverfahrens hauptsächlich in der Radiodiagnostik verfolgt.

Heptahelikale Rezeptoren sind Zielmoleküle von vielen pharmakologischen Wirkstoffen (z. B. β-Blocker, H2-Säure-Blocker, Antihistaminika). Neben therapeutischen Ansätzen werden hauptsächlich radioaktiv markierte agonistische Liganden dieser Rezeptoren diagnostisch für die sog. Rezeptorszintigraphie zur In-vivo-Detektion und Lokalisation von Tumoren eingesetzt. Hierbei wird der Mechanismus der rezeptorvermittelten Endozytose, z. B. durch den Somatostatinrezeptor, der auf neuroendokrinen Tumoren vermehrt exprimiert ist, ausgenutzt. Klinisch zugelassen für die szintigraphische Routinediagnostik ist das Somatostatinanalogon ¹¹¹In-DTPA-pentetreotid (Octreoscan®); Literatur: J. Steroid Biochem. Mol. Biol. 37, 1079-82, 1990, J. Nucl. Med. 32, 1184-9, 1991,; J. Nucl. Med. 33, 652-8, 1992; Digestion 3, 54-9, 1994, J. Clin. Invest. 93, 1321-5, 1994, Metabolism 45, 21-3, 1996.

Ein anderer Ansatz besteht in der Nutzung von radioaktiv markiertem VIP und VIP-Analoga, welche an den VIP-Rezeptor binden. Der VIP-Rezeptor wird von einem breiten Spektrum von Tumoren vermehrt exprimiert (u. a. Adenokarzinome).
WO 96/30055 beschreibt radiodiagnostische und radiotherapeutische Reagenzien, speziell VIP-rezeptorbindende Peptide, die radioaktiv markiert sind und für die Radiodiagnostik und -therapie eingesetzt werden können. Mit besonderem Vorteil beschrieben sind VIP-rezeptorbindende, mit Tc-99m markierbare Peptide für die Szintigraphie. Weitere Literatur: Cancer Research 54, 690-700, 1994; Endocrinology 136, 2662-80, 1994, J. Nucl. Med. 40, 353-361, 1999.

Alle beschriebenen diagnostischen Ansätze, die auf dem Somatostatinrezeptor und VIP-Rezeptor basieren, sind radiodiagnostische Ansätze (Szintigraphie mit ¹²³I, ¹²⁵I, ¹¹¹In oder ^{99m}Tc-markierten Peptiden).
Literatur: EP 588754, US 5650134; US 5620675; US 5225180; WO 96/23527; J. Steroid Biochem. Mol. Biol. 37, 1083-87, 1990; Lancet 242-4, 1989, J. Nucl. Med. 39, 1913-17, 1998.

Bisher sind jedoch keine fluoreszenzmarkierten Peptide bekannt, die mit Farbstoffen konjugiert sind, die eine In-vivo-Fluoreszenzdetektion von Tumoren ermöglichen (Photochem. Photobiol. 68, 603-632, 1998).

Der Erfindung liegt die Aufgabe zu Grunde, neue Verbindungen zur Verfügung zu stellen, die eine sensitive Diagnose von Tumoren durch Detektion von Fluoreszenzstrahlung unter Ausnutzung einer rezeptorspezifischen Bindung der Verbindungen an das Zielgewebe ermöglichen. Hierbei sollen spezielle Farbstoffmoleküle, die an Biomoleküle gekoppelt sind, ein hochsensitiv detektierbares Fluoreszenzsignal liefern.

Die Aufgabe wird durch die Bereitstellung von Verbindungen, die Fluoreszenzfarbstoffe enthalten, welche kovalent an kurzkettige Peptide gekoppelt sind, gelöst. Diese Konjugate besitzen eine hohe Bindungsaffinität zu heptahelikalen Rezeptoren, speziell dem Somatostatinrezeptor, dem VIP-Rezeptor (Vasoaktives intestinales Peptid) und dem Neurotensinrezeptor, und werden ggf. durch rezeptorvermittelte Endozytose intrazellulär aufgenommen. Die erfindungsgemäßen Verbindungen sind daher geeignet für die technisch einfache, unschädliche optische Diagnostik von Tumorzellen und Tumorgeweben, die Somatostatinrezeptoren, VIP-Rezeptoren oder Neurotensinrezeptoren im Vergleich zu gesunden Zellen erhöht exprimieren. Insbesondere geeignet sind die Verbindungen für die Fluoreszenzdiagnostik und mit besonderem Vorteil für die fluoreszenzendoskopische Diagnostik in Hohlorganen, wie dem Ösophagus, der Zervix, des Kolons und der Bronchien von verschiedenen Tumortypen, wie z. B. Adenokarzinomen, neuroendokrinen Tumoren oder duktalen pankreatischen Tumoren.

Besonders bevorzugte Farbstoffe zeichnen sich dadurch aus, daß sie bestimmte photophysikalische und chemische Anforderungen erfüllen. Aus photophysikalischer Sicht müssen die Farbstoffe hohe Absorptionskoeffizienten und hohe Fluoreszenzquantenausbeuten besitzen, um ein effektives Signal auch bei geringsten Gewebekonzentrationen zu erzeugen. Die Absorptionsmaxima müssen frei wählbar einem weiten spektralen Bereich überdecken. So ist für die Detektion in tieferen Gewebeschichten (mehrere Zentimeter unter der Oberfläche) der Spektralbereich zwischen 600 und 900 nm essentiell, während für oberflächliche Detektion Absorptionswellenlängen von 400 bis 600 nm ausreichen. Aus chemischer Sicht müssen die Farbstoffe eine hohe Photostabilität besitzen und keine Zersetzungserscheinungen (Photobleaching) während der Anregung aufweisen. Die Farbstoffe müssen als Synthesebaustein in der festphasensynthetischen Herstellung der Peptide einsetzbar sein, und somit unter den gängigen Synthesebedingungen stabil sein, damit eine einfache, günstige Produktion strukturell definierter Farbstoff-Peptid-Konjugate mit festem stöchiometrischen Verhältnis zwischen Farbstoff und Peptid gewährleistet ist. Die Anforderungen werden am besten von Polymethinfarbstoffen, insbesondere Cyanin-, Merocyanin-, Oxonol- und Squariliumfarbstoffen erfüllt.

Gegenstand der Erfindung sind daher Cyaninfarbstoffe die diesen Anforderungen genügen.

Aus der Klasse der Polymethinfarbstoffe sind die Cyaninfarbstoffe, z. B. die auf der Indolstruktur basierenden Indocarbo-, Indodicarbo- und Indotricarbocyanine besonders vorteilhaft. Diese Strukturen zeichnen sich durch eine hohe chemische und photochemische Stabilität aus. Durch günstige Synthese sind Derivate erhältlich, die beliebig zwischen 400 und 1000 nm absorbieren und fluoreszieren, durch Substitution mit geeigneten Linkern und funktionellen Gruppen, vorzugsweise Carboxylgruppen, an Peptide gekoppelt werden können und eine hohe Wasserlöslichkeit, vorzugsweise durch Sulfonatgruppen, besitzen. Im Gegensatz zu literaturbekannten Cyaninfarbstoffen besitzen die erfindungsgemäß verwendeten Verbindungen nur eine reaktive Gruppe, die eine stöchiometrisch definierte Kopplung an das Peptid im Zuge der Harzsynthese des Konjugates ermöglicht.

Besonders bevorzugte Ausführungsformen der erfindungsgemäßen Verbindungen der allgemeinen Formel I zeichnen sich daher dadurch aus,
- daß das Farbstoffmolekül A¹ und/oder A² für einen Indocarbocyanin-, einen Indodicarbocyanin- oder einen Indotricarbocyaninfarbstoff steht,
- daß das Farbstoffmolekül A¹ und/oder A² für einen Indocarbocyanin-, einen Indodicarbocyanin- oder einen Indotricarbocyaninfarbstoff der allgemeinen Formel XIII oder XIV 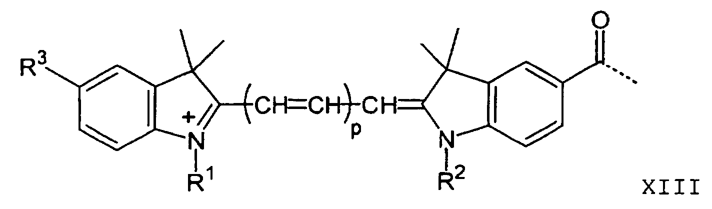 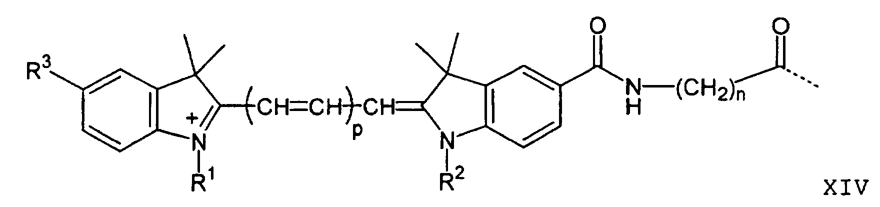
worin
p für 1, 2 oder 3,
n für eine Zahl von 1, 2, 3, 4 oder 10 steht,
R¹ und R² unabhängig voneinander für einen 4-Sulfobutyl-, 3-Sulfopropyl-, 2-Sulfoethyl-, 3-Methyl-3-sulfopropyl-, Methyl-, Ethyl- oder Propylrest stehen, und
R³ für Wasserstoff, ein Chlor-, Brom-, Iodatom oder eine Nitrogruppe oder für einen Rest -COOE¹, -CONE¹E², -NHCOE¹, -NHCONHE¹, -NE¹E², -OE¹, -OSO₃E¹, -SO₃E¹, -SO₂NHE¹,
wobei E¹ und E² unabhängig voneinander für ein Wasserstoffatom oder für einen Methyl-, Ethyl- oder einen C₃-C₆-Alkylrest, der von 0 bis 2 Sauerstoffatomen und/oder von 0 bis 1 Carbonylgruppen unterbrochen ist und/oder mit 0 bis 5 Hydroxygruppen substituiert ist, oder für einen Poly(oxyethylen)glykolrest mit 2 bis 30 -CH₂CH₂O-Einheiten stehen,
steht,
- daß das Farbstoffmolekül A¹ und/oder A² für einen Indocarbocyanin-, einen Indodicarbocyanin- oder einen Indotricarbocyaninfarbstoff der allgemeinen Formel XIII oder XIV 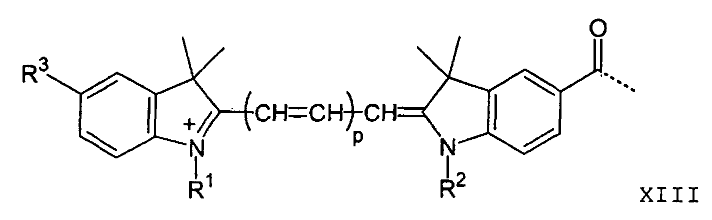 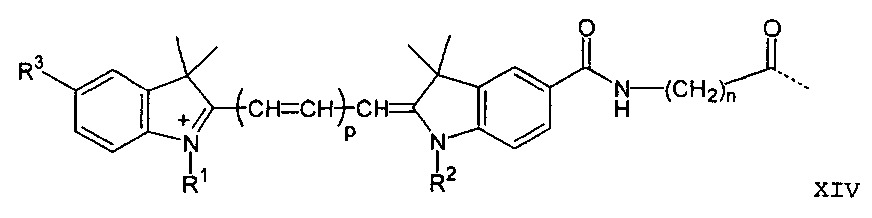
worin
p für 1, 2 oder 3,
n für 1, 2 oder 4,
R¹ und R² unabhängig voneinander für einen 4-Sulfobutyl- oder 3-Sulfopropylrest,
R³ für Wasserstoff oder für einen Rest -COOE¹ oder -CONHE¹,
wobei E¹ ein Wasserstoffatom oder ein Methyl-, Ethyloder ein C₃-C₆-Alkylrest, der von '0 bis 2 Sauerstoffatomen und/oder von 0 bis 1 Carbonylgruppen unterbrochen ist und/oder mit 0 bis 5 Hydroxygruppen substituiert ist, bedeutet,
steht,
- daß das Farbstoffmolekül A¹ und/oder A² für einen Indotricarbocyaninfarbstoff der allgemeinen Formel XV oder XVI steht: 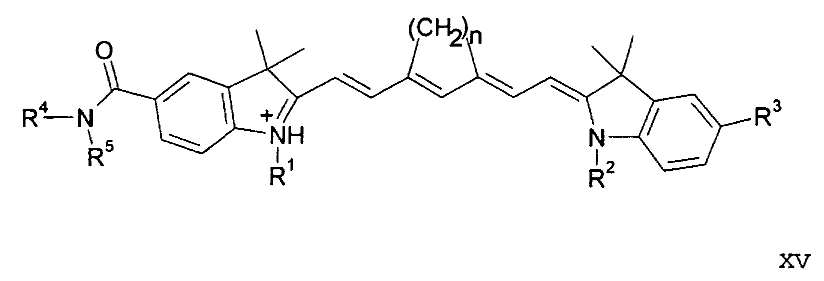 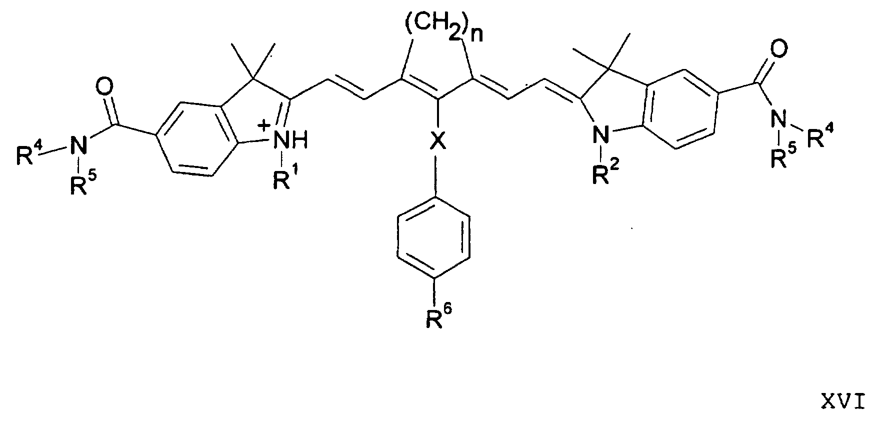
worin
n für 2 oder 3 steht,
R¹ und R² unabhängig voneinander einen 4-Sulfobutyl-, 3-Sulfopropyl- oder 2-Sulfoethylrest darstellen,
R³ für einen Rest -CONH-Peptid, -CONH-(CH₂)ₘ-CONH-Peptid, - CONH-(CH₂)ₙ-NH-CS-NH-Peptid oder -CONH-(CH₂)ₙ-NHCO-CH₂-Peptid mit m = 1 bis 10 und n = 2 oder 3 steht,
oder eine folgende Gruppe darstellt: 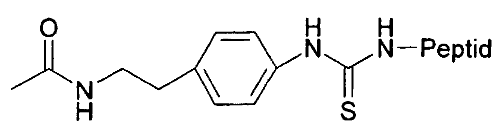 R⁴ und R⁵ unabhängig voneinander für ein Wasserstoffatom, einen Methylrest oder einen hydroxylierten Alkylrest wie z.B. 2-Hydroxyethyl, 3-Hydroxypropyl, 2,3-Dihydroxypropyl, 1,3-Dihydroxy-2-propyl, 2,3,4-Trihydroxybutyl, 1,3,4-Trihydroxy-2-butyl, 2,3,4,5,6-Pentahydroxyhexyl stehen,
R⁶ für eine der folgenden Gruppen steht:
-(CH₂)ₘ-CONH-Peptid mit m = 0 bis 2,
-(CH₂)ₘ-NH-CS-NH-Peptid mit m = 0 bis 2,
und X für ein Sauerstoffatom oder ein Schwefelatom steht;
- daß das Farbstoffmolekül A¹ und/oder A² für einen Indotricarbocyaninfarbstoff der allgemeinen Formel XVII steht: 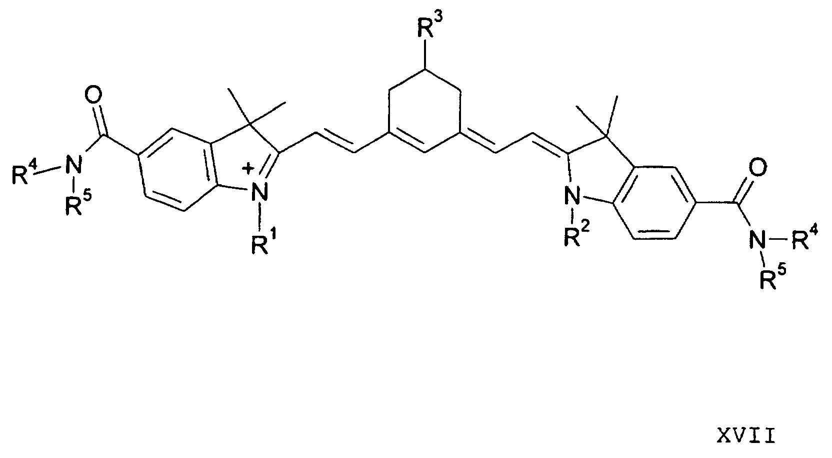
worin
R¹ und R² unabhängig voneinander für einen 4-Sulfobutyloder 3-Sulfopropylrest darstellen,
R³ für einen Rest -CONH-Peptid, -CONH-(CH₂)ₘ-CONH-Peptid, -CONH-(CH₂)ₙ-NH-CS-NH-Peptid oder -CONH-(CH₂)ₙ-NHCO-CH₂-Peptid mit m = 1 bis 10 und n = 2 oder 3 steht,
oder eine folgende Gruppe darstellt: 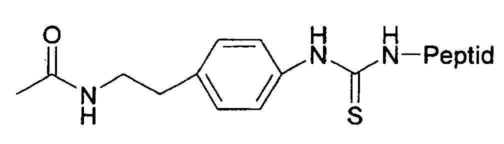 und R⁴ und R⁵ unabhängig voneinander für ein Wasserstoffatom, einen Methylrest oder einen hydroxylierten Alkylrest wie z.B. 2-Hydroxyethyl, 3-Hydroxypropyl, 2,3-Dihydroxypropyl, 1,3-Dihydroxy-2-propyl, 2,3,4-Trihydroxybutyl, 1,3,4-Trihydroxy-2-butyl, 2,3,4,5,6-Pentahydroxyhexyl stehen.

Die Substanzen markiert mit den erfindungsgemäßen Cyaninfarbstoffen haben verschiedene Vorteile gegenüber radioaktiv markierten Substanzen. Fluor-eszenzfarbstoffe können beliebig oft zur Fluoreszenzemission angeregt werden. Es liegt kein kontinuierliches Signal vor, das einem Zerfall mit entsprechender Halbwertszeit zu Grunde liegt. Dementsprechend ist der Zeitpunkt der Diagnose beliebig wählbar und beliebig oft wiederholbar und nicht durch die Halbwertszeit eines Isotops limitiert. Der Patient ist keiner ionisierenden Strahlung ausgesetzt, die verwendete Lichtstrahlung ist in den verwendeten Dosen unschädlich. Die optische Detektionstechnik erlaubt die hochsensitive Detektion weniger Photonen und ist daher bezüglich der Sensitivität mit der Radio-diagnostik vergleichbar.

Ein Verfahren zur Diagnostik mittels Nahinfrarotstrahlung (NIR-Strahlung) unter Verwendung von Farbstoffbiomolekülkonjugaten wurde beschrieben (WO 96/17628). Als besonders vorteilhaft erwies sich im Falle der vorliegenden Erfindung, daß die Peptidkonjugate in günstiger Ausbeute und hoher Reinheit durch das Verfahren der automatisierten Festphasensynthese darstellbar sind. Überraschenderweise wurde gefunden, daß verschiedene Carboxylgruppentragende Indocyaninverbindungen als Aminosäureanaloga eingesetzt und sowohl N-terminal als auch an eine Aminogruppe des Lysins an der Festphase (Harz) gekoppelt werden können. Nach Abspaltung vom Harz und chromatographischer Reinigung wurden die Farbstoff-Peptid-Konjugate in Reinheiten > 95% erhalten. Ein neues Kopplungsverfahren erlaubt die Kopplung von Halogenacetyl-Farbstoffen an die Aminosäure Cystein oder Homocystein, die eine beliebige Aminosäure der nativen VIP-Sequenz ersetzen kann.

Das wesentliche Problem bei Nutzung von Licht zur Fluoreszenzanregung ist die begrenzte Eindringtiefe des Lichtes, die im VIS im Submillimeterbereich liegt, im NIR jedoch Zentimeter betragen kann. Hinsichtlich der Eindringtiefe unproblematisch sind Detektionsverfahren in oberflächlichen Gewebeerkrankungen, sowie weichen Geweben. Erfindungsgegenstand sind daher mammographische Verfahren, endoskopische Verfahren und intraoperative Verfahren, bei denen unter Verwendung der Farbstoff-Peptid-Konjugate enthaltend die erfindungsgemäßen Verbindungen erkrankte Gewebebereiche durch Detektion der Fluoreszenz oder der nicht absorbierten Strahlung diagnostiziert werden. Besonderer Erfindungsgegenstand ist die Verwendung der Farbstoff-Peptid-Konjugate enthaltend die erfindungsgemäßen Verbindungen in endoskopischen Verfahren, z. B. die Koloskopie, Bronchoskopie, Oesophagealendoskopie, bei denen oberflächennahe Gewebeveränderungen diagnostiziert werden. Die Verwendung von Weißlicht mit direkter visueller Beurteilung ist in der endoskopischen Diagnostik weit verbreitet. Die erfindungsgemäßen Verbindungen tragen durch die Erzeugung eines gewebespezifischen Signals zu einer entscheidenden Verbesserung des Verfahrens bei, insbesondere bei der Diagnostik frühzeitiger, visuell nicht erfaßbarer Gewebeveränderungen (z. B. Dysplasien des Kolons).

Es wurde gefunden, daß sich nach Versprühen der erfindungsgemäßen farbstoffgekoppelten Verbindungen im Darm von Ratten mit chemisch induzierten Dysplasien und Kolonkarzinomen, anschließendem Spülen und der Durchführung einer endoskopischen Fluoreszenzdiagnostik (Anregung 740 nm, Detektion oberhalb 760 nm) Gewebebereiche erhöhter Fluoresenz im Kolon nachweisen ließen.

Gegenstand der Erfindung ist daher auch ein Verfahren zur endoskopischen Fluoreszenzdiagnostik, insbesondere des Gastrointestinaltraktes, unter Verwendung der erfindungsgemäßen Verbindungen. Dabei wird dem Gewebe eine oder mehrere der Substanzen, vorzugsweise intravenös, oder topisch durch Versprühen zugeführt und Licht aus dem entsprechenden Spektralbereich zur elektronischen Anregung des verwendeten Farbstoffes eingestrahlt. Die reflektierte oder die vom Farbstoff emittierte Fluoreszenzstrahlung wird registriert. Bevorzugt sind die Methoden, bei denen das Gewebe großflächig bestrahlt und die Fluoreszenzstrahlung örtlich aufgelöst durch Aufnahme mit einer CCD-Kamera dargestellt wird oder die abzubildenden Gewebeareale mit einem Lichtleiter abgerastert und die erhaltenen Signale rechnerisch in ein synthetisches Bild umgesetzt werden. Dabei kann die Fluoreszenz spektral und/oder phasenselektiv sowie stationär und/oder zeitaufgelöst detektiert und ausgewertet werden. Die erhaltenen Fluoreszenzbilder können simultan mit Weißlichtbildern erzeugt werden und zur Datenauswertung in einer Abbildung übereinander dargestellt werden.

Die Synthese der erfindungsgemäßen Verbindungen erfolgt in Anlehnung an literaturbekannte Methoden. Die Peptide werden festphasensynthetisch an Polymerharzen hergestellt. Einzelheiten sind dem Fachmann bekannt.
Literatur: Peptide chemistry - A practical textbook (M. Bodanszky), 2nd edition, Springer-Verlag Heidelberg 1993; Anti-Cancer Drug Design 12, 145-167, 1997; J. Am. Chem. Soc. 117, 11821-2, 1995.

Die Farbstoffe werden separat hergestellt und dann im Zuge der festphasensynthetischen Darstellung der Peptide an diese gekoppelt und die erfindungsgemäßen Verbindungen nach Abspaltung vom Harz und Reinigung als hochreine Verbindungen erhalten. Bevorzugt sind solche Farbstoffe, die Carboxylgruppen enthalten, welche nach Aktivierung mittels gängiger Reagenzien mit Aminogruppen des Peptides, insbesondere der ε-Aminogruppe des Lysins oder des N-terminalen Peptidaminogruppe, gekoppelt werden. Weiterhin bevorzugt sind Farbstoffe mit Halogenalkyl- oder Halogenacetylresten, die an Thiolgruppen des Peptides, insbesondere der Aminosäure Cystein oder Homocystein, gekoppelt werden.
Literatur zur Synthese von Polymethinfarbstoffen: Bioconjugate Chem. 4, 105-111, 1993; Bioconjugate Chem. 7, 356-62, 1996; Bioconjugate Chem. 8, 751-56, 1997; Cytometry 10, 11-19, 1989 und 11, 418-30, 1990; J. Heterocycl. Chem. 33, 1871-6, 1996; J. Org. Chem. 60, 2391-5, 1995; Dyes and Pigments 17, 19-27, 1991, Dyes and Pigments 21, 227-34, 1993; J. Fluoresc. 3, 153-155, 1993; Anal. Biochem. 217, 197-204, 1994; US 4981977; US 5688966; US 5808044; WO 97/42976; WO 97/42978; WO 98/22146; WO 98/26077; EP 0800831.

Besonders geeignet für eine Kopplung an Peptide an fester Phase sind Farbstoffe, die genau eine Carboxylgruppe enthalten, mit besonderem Vorteil Cyaninfarbstoffe der allgemeinen Formel XVIII 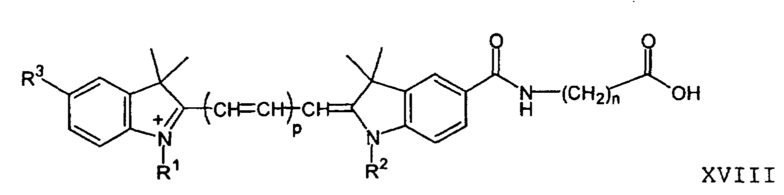 worin
p für 1, 2 oder 3,
n für 1, 2, 3, 4 oder 10 steht,
R¹ und R² unabhängig voneinander für einen 4-Sulfobutyl-, 3-Sulfopropyl-, 2-Sulfoethyl-, 3-Methyl-3-sulfopropyl-, Methyl-, Ethyl- oder Propylrest stehen, und
R³ für Wasserstoff oder für einen Rest -COOE¹, -CONE¹E², -NHCOE¹, -NHCONHE¹, -NE¹E², -OE¹, -OSO₃E¹, -SO₃E¹, -SO₂NHE¹,
wobei E¹ und E² unabhängig voneinander für ein Wasserstoffatom oder für einen Methyl-, Ethyl- oder einen C₃-C₆-Alkylrest, der von 0 bis 2 Sauerstoffatomen und/oder von 0 bis 1 Carbonylgruppen unterbrochen ist und/oder mit 0 bis 5 Hydroxygruppen substituiert ist, stehen,
steht,
oder Cyaninfarbstoffe der allgemeinen Formel XIX oder XX 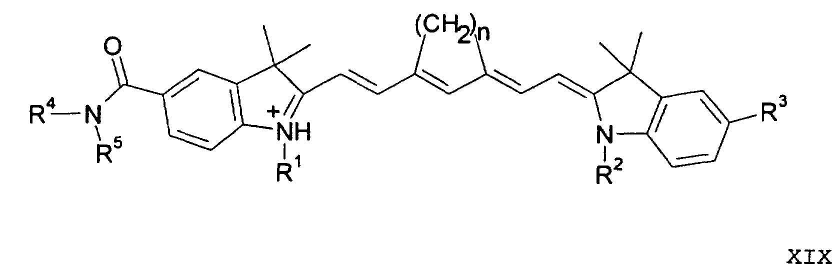 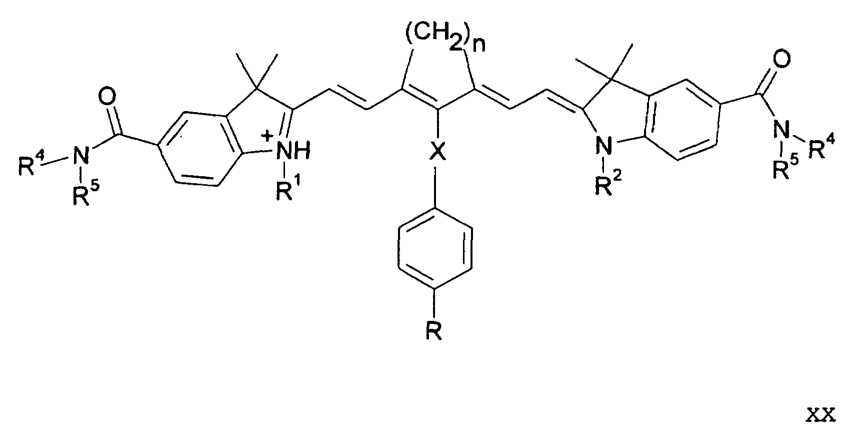 worin
n für 2 oder 3 steht,
R¹ und R² unabhängig voneinander einen 4-Sulfobutyl-, 3-Sulfopropyl- oder 2-Sulfoethylrest darstellen,
R³ für eine -COOH-Gruppe oder einen der folgenden Reste steht:
-CONH-(CH₂)ₙ-COOH mit n = 2 oder 3,
-CONH-(CH₂)ₙ-NCS mit n = 2 oder 3,
-CONH-(CH₂)ₙ-NHCO-CH₂-X¹ mit n = 2 oder 3 und X¹ = Cl, Br, I 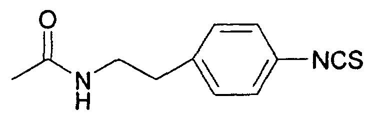
R⁴ und R⁵ unabhängig voneinander für ein Wasserstoffatom, einen Methylrest oder einen hydroxylierten Alkylrest wie z.B. 2-Hydroxyethyl, 3-Hydroxypropyl, 2,3-Dihydroxypropyl, 1,3-Dihydroxy-2-propyl, 2,3,4-Trihydroxybutyl, 1,3,4-Trihydroxy-2-butyl, 2,3,4,5,6-Pentahydroxyhexyl stehen,
R⁶ für eine der folgenden Gruppen steht:
- (CH₂)ₘ-COOH mit m = 0 bis 2,
- (CH₂)ₘ-NCS mit m = 0 bis 2,
und X für ein Sauerstoffatom oder ein Schwefelatom steht;
oder Cyaninfarbstoffe der allgemeinen Formel XXI 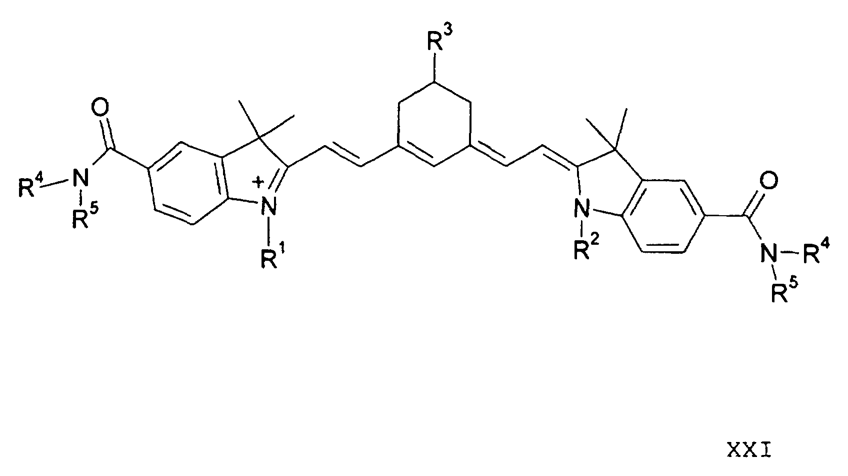 worin
R¹ und R² unabhängig voneinander für einen 4-Sulfobutyl-, 3-Sulfopropyl- oder 2-Sulfoethylrest darstellen,
R³ für eine -COOH-Gruppe oder einen der folgenden Reste steht:
-CONH-(CH₂)ₙ-COOH mit n = 2 oder 3,
-CONH-(CH₂)ₙ-NCS mit n = 2 oder 3,
-CONH-(CH₂)ₙ-NHCO-CH₂-X¹ mit n = 2 oder 3 und X¹ = Cl, Br, I 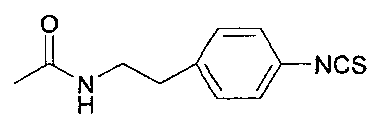
und R⁴ und R⁵ unabhängig voneinander für ein Wasserstoffatom, einen Methylrest oder einen hydroxylierten Alkylrest wie z.B. 2-Hydroxyethyl, 3-Hydroxypropyl, 2,3-Dihydroxypropyl, 1,3-Dihydroxy-2-propyl, 2,3,4-Trihydroxybutyl, 1,3,4-Trihydroxy-2-butyl, 2,3,4,5,6-Pentahydroxyhexyl stehen.

Der Vorteil nur einer aktivierbaren Gruppe, wie z.B. einer Carboxylgruppe, oder einer bereits aktivierten Gruppe, wie z.B. einem Isothiocyanat, einer Halogenalkylgruppe oder einer Halogenacetylgruppe, besteht darin, daß eine chemisch einheitliche Kopplung erfolgen kann. Die Halogenacetylgruppe hat den besonderen Vorteil, daß eine chemisch einheitliche Kopplung an die Mercaptogruppe des Cysteins oder Homocysteins erfolgt. Diese Kopplung kann in Lösung an das ungebundene und von Schutzgruppen befreite Peptid erfolgen. Durch die aktivierten Gruppen ist eine Kopplung an Peptide möglich, ohne daß Nebenreaktionen auftreten. Zur Erhöhung der Wasserlöslichkeit weisen die Peptid-Farbstoffkonjugate am Farbstoff eine erhöhte Anzahl von Hydroxygruppen auf. Durch die Position des Linkers am Indolsystem des Farbstoffes kann zusätzlich eine ausreichende Hydrophilie durch Sulfonatgruppen enthaltende Reste an den Stickstoffatomen des Indolsystems erzeugt werden. Dadurch kann eine strukturell einheitliche Kopplungsreaktion mit den Peptiden (siehe Beispiele 4 bis 38 und 44 bis 49) durchgeführt werden.

Folgende Beispiele erläutern die Erfindung:

### Beispiele 1 bis 3: Synthese der Indocyaninfarbstoffe 1-3 zur festphasensynthetischen Kopplung an Aminogruppen (N-terminal oder ε-Lysin) der Peptide

Die Synthese erfolgt generell ausgehend von 1-(4-Sulfobutyl)-2,3,3-trimethyl-*3H*-indolenin und 1-(4-Sulfobutyl)-2,3,3-trimethyl-5-carboxy-*3H*-indolenin (Cytometry 10, 11-19, 1989, Talanta 39, 505-510, 1992).

### Beispiel 1: Synthese von 1,1'-Bis(4-sulfobutyl)indocarbocyanin-5-carbonsäure, Natriumsalz (1)

0,8 g ( 4,0 mmol) N,N-Diphenylformamidin werden in 15 ml Essigsäureanhydrid vorgelegt und bei Raumtemp. portionsweise mit 1,4 g (4,2 mmol) 1-(4-Sulfobutyl)-2,3,3-trimethyl-5-carboxy-*3H*-indolenin versetzt, 30 min bei 120 °C gerührt und dann mit einem Wasserbad auf Raumtemp. abgekühlt. Anschließend werden 1,2 g (4,1 mmol) 1-(4-Sulfobutyl)-2,3,3-trimethyl-*3H*-indolenin, 1,2 g (14,6 mmol) wasserfreies Natriumacetat, 15 ml Essigsäureanhydrid und 6 ml Essigsäure zugegeben. Das Reaktionsgemisch wird 1 h auf 120 °C erhitzt, die tiefrote Lösung abgekühlt und mit 100 ml Ether versetzt. Der ausgefallene Feststoff wird abfiltriert. Es erfolgt eine chromatographische Reinigung an RP-Kieselgel EUROPREP 60-30 C18, 60A, 20-45 m (Eluens: Wasser/MeOH, Stufengradient von 0 % auf 70 % MeOH). Die produktenthaltenden Fraktionen werden am Rotationsverdampfer vom Methanol befreit und anschließend lyophilisiert, Ausbeute: 1,5 g (58 %), rotes Lyophilisat.

### Beispiel 2:

### Synthese von 1,1'-Bis(4-sulfobutyl)indodicarbocyanin-5-carbonsäure, Natriumsalz (2)

1,2 g (4,1 mmol) 1-(4-Sulfobutyl)-2,3,3-trimethyl-*3H*indolenin und 1,0 g ( 3,9 mmol) Malonaldehyd-bis-phenylimin-hydrochlorid werden in 15 ml Essigsäureanhydrid 30 min bei 120 °C gerührt und dann mit einem Wasserbad auf Raumtemp. abgekühlt. Anschließend werden nacheinander 1,4 g (4,2 mmol) 1-(4-Sulfobutyl)-2,3,3-trimethyl-5-carboxy-*3H*-indolenin, 1,2 g (14,6 mmol) wasserfreies Natriumacetat, 15 ml Essigsäureanhydrid und 6 ml Essigsäure zugegeben. Das Reaktionsgemisch wird 1 h auf 120 °C erhitzt, die nun blaue Lösung abgekühlt und mit 100 ml Ether versetzt. Die Aufarbeitung und Reinigung erfolgt wie in Beispiel 1 beschrieben, Ausbeute: 1,8 g (66 %), blaues Lyophilisat.

### Beispiel 3:

### Synthese von 1,1'-Bis(4-sulfobutyl)indotricarbocyanin-5-carbonsäure, Natriumsalz (3)

1,2 g (4,1 mmol) 1-(4-Sulfobutyl)-2,3,3-trimethyl-*3H*indolenin und 1,1 g ( 3,9 mmol) Glutaconaldehyddianilhydrochlorid werden in 15 ml Essigsäureanhydrid 30 min bei 120 °C gerührt und dann mit einem Wasserbad auf Raumtemp. abgekühlt. Anschließend werden 1,4 g (4,2 mmol) 1-(4-Sulfobutyl)-2,3,3-trimethyl-5-carboxy-*3H*indolenin, 1,2 g (14,6 mmol) wasserfreies Natriumacetat, 15 ml Essigsäureanhydrid und 6 ml Essigsäure zugegeben. Das Reaktionsgemisch wird 1 h auf 120 °C erhitzt, die nun blaue Lösung abgekühlt und mit 100 ml Ether versetzt. Die Aufarbeitung und Reinigung erfolgt wie in Beispiel 1 beschrieben, Ausbeute: 1,8 g (60 %), blaues Lyophilisat.

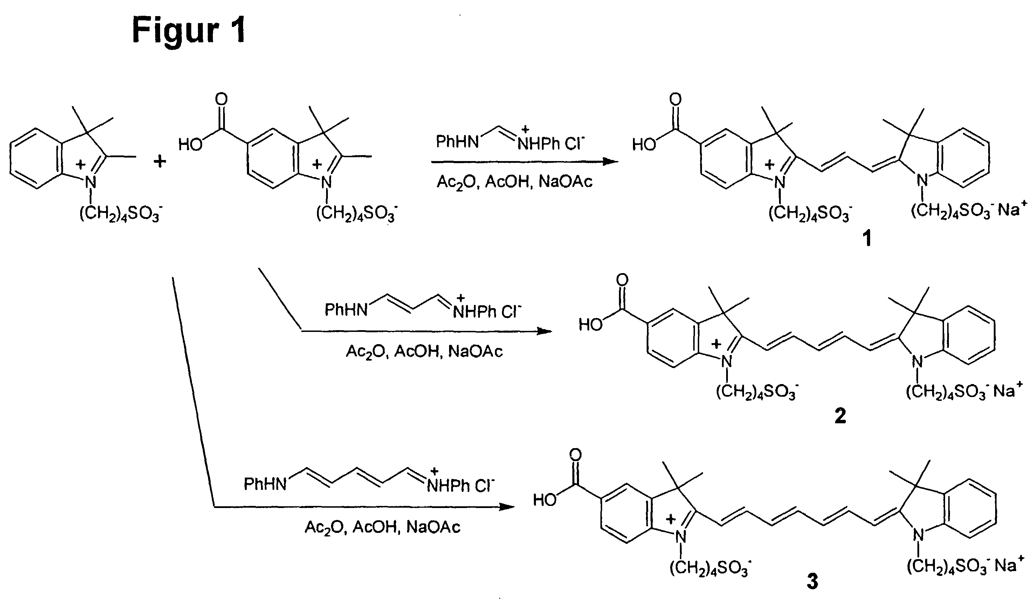

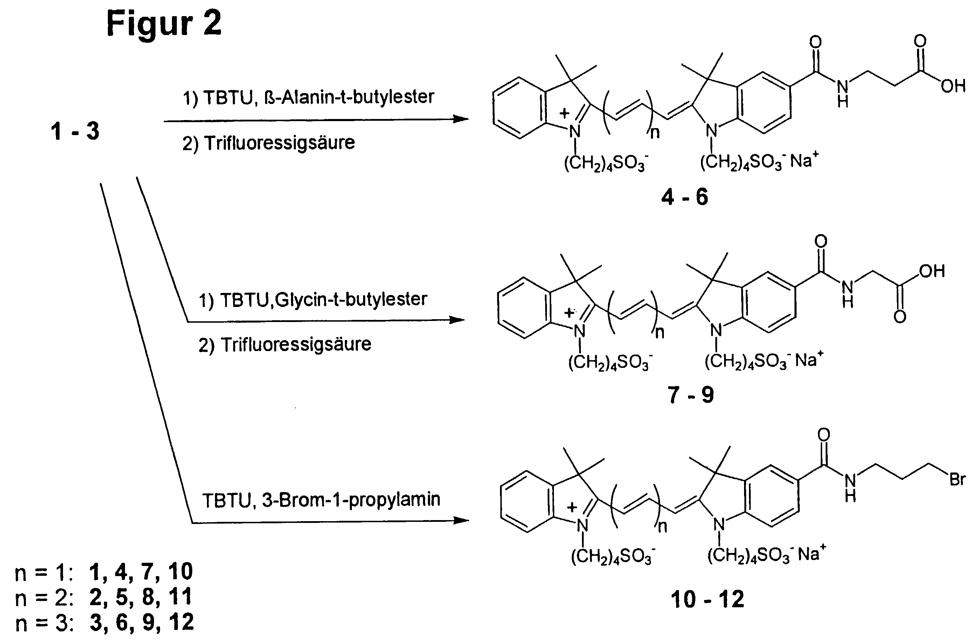

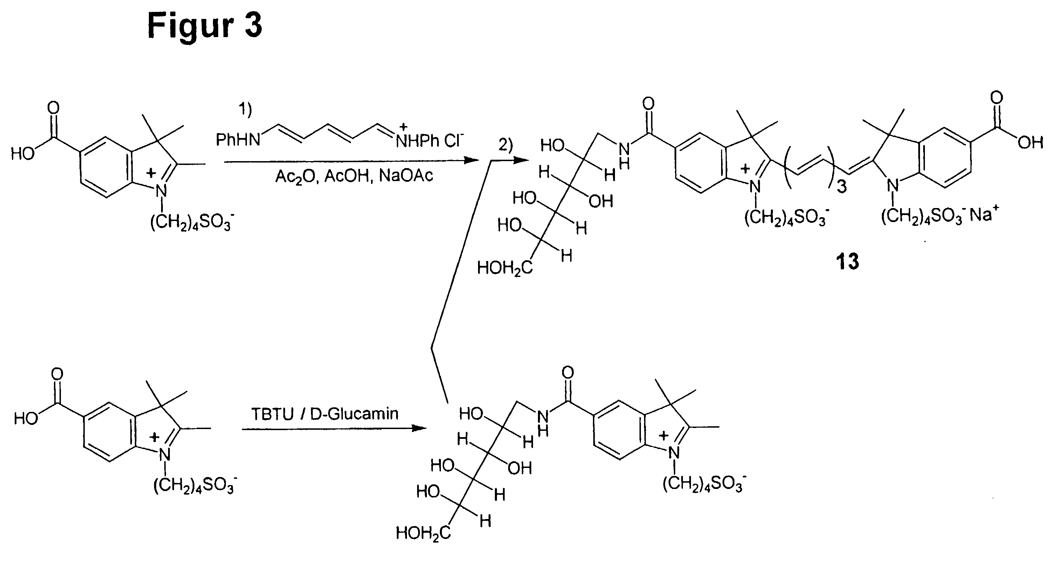

Die Strukturen der Verbindungen aus Beispiel 1-3 sind in Fig. 1 dargestellt.

### Beispiele 4 bis 6: Synthese der Indocyaninfarbstoffe 4-6 aus 1-3 zur festphasensynthetischen Kopplung an Aminogruppen (N-terminal oder ε-Lysin) der Peptide

Die Synthese erfolgt durch Amidierung der Farbstoffe 1-3 mit β-Alanin-t-butylester und saurer Spaltung der t-Butylestergruppe.

Eine Lösung von 0,5 mmol des Farbstoffes **1-3** und 0,1 g (1,0 mmol) Triethylamin in 20 ml Dimethylformamid wird bei 0 °C mit 0,5 mmol TBTU in 10 ml Dimethylformamid versetzt und 15 min bei 0 °C gerührt. Anschließend wird eine Lösung von 0,11 g (0,6 mmol) β-Alanin-t-butylesterhydrochlorid und 0,6 mmol Triethylamin in 5 ml Dimethylformamid zugetropft und das Reaktionsgemisch 2 h bei Raumtemp. gerührt. Nach Zugabe von 100 ml Diethylether wird der ausgefallene Feststoff abfiltriert, in 20 ml Dichlormethan gelöst, mit 10 ml Trifluoressigsäure versetzt und 24 h bei Raumtemp. gerührt. Das Gemisch wird im Vakuum eingeengt und der Rückstand wie in Beispiel 1 beschrieben chromatographisch gereinigt und lyophilisiert; Ausbeuten: 0,21 g (58%) **4,** 0,29 g (76%) **5,** 0,28 g (72%) **6.**

### Beispiele 7 bis 9: Synthese der Indocyaninfarbstoffe 7-9 aus 1-3 zur festphasensynthetischen Kopplung an Aminogruppen (N-terminal oder ε-Lysin) der Peptide

Die Darstellung erfolgt analog der Beispiele 4-6 unter Verwendung von 0,1 g (0,6 mmol) Glycin-t-butylesterhydrochlorid, Ausbeuten: 0,25 g (68%) **4,** 0,30 g (80%) **5,** 0,32 g (83%) **6.**

### Beispiele 10 bis 12: Synthese der Indocyaninfarbstoffe 10-12 aus 1-3 zur festphasensynthetischen Kopplung an Peptide durch Kopplung an Thiolgruppen von Cystein.

Die Synthese erfolgt durch Amidierung der Farbstoffe 1-3 mit 3-Aminopropanol und anschließender Überführung der Alkoholgruppe in ein Bromid.

Eine Lösung von 0,5 mmol des Farbstoffes **1-3** und 0,1 g (1,0 mmol) Triethylamin in 20 ml Dimethylformamid wird bei 0 °C mit 0,5 mmol TBTU in 10 ml Dimethylformamid versetzt und 15 min bei 0 °C gerührt. Anschließend wird eine Lösung von 850 mg (1,2 mmol) 3-Aminopropanol und 1,2 mmol Triethylamin in 5 ml Dimethylformamid zugetropft und das Reaktionsgemisch 6 h bei Raumtemp. gerührt. Nach Zugabe von 100 ml Diethylether wird der ausgefallene Feststoff abfiltriert und wie in Beispiel 1 beschrieben chromatographisch gereinigt und lyophilisiert.
Die Umsetzung zu den Bromiden **10-12** erfolgt durch Rühren von 0,3 mmol der Zwischenprodukte mit 55 mg (0,5 mmol) N-Bromsuccinimid und 130 mg (0,5 mmol) Triphenylphosphin in einem Gemisch von 4 ml Dichlormethan und 4 ml Dimethylformamid für 48 h bei 4 °C. Durch Zugabe von 3 ml Ether werden die Produkte ausgefällt, abfiltriert und als Rohprodukte in der Peptidkopplung eingesetzt.

### Beipiele 14-16 und 18-27: Harzsynthese von Peptidkonjugaten aus VIP-rezeptorbindenden Peptiden und den Farbstoffen 1-9 und 13.

*a) Festphasenpeptidsynthese*: An 50 µmol TentaGel-Sram-Harz (Rapp Polymere, Tübingen) werden die Peptide nach der Fmoc-Strategie analog dem Standard-Fmoc-Maschinen-Protokoll (Pept. Res., 36 (1990) 225) unter Verwendung des Kupplungsreagenzes PyBOP (Benzotriazol-1-yloxy-trispyrrolidionophosphonium hexafluorophosphat) und N-Methylmorpholin synthetisiert. Die folgenden Seitenschutzgruppen wurden benutzt: Trityl für Cys, His, Asn und Gln; *t*-Butyl für Asp, Glu, Ser und Thr; *t*-butyloxycarbonyl für Lys und Trp und Pentamethylchromansulfonyl für Arg.
   Die Kopplung an die ε-Aminogruppe eines Lysins (Beispiel 24) wird durch orthogonale Schutzgruppentechnik erzielt. Als Lysinbaustein wird Fmoc-Lys(Dde)-OH eingesetzt und das Peptid wie oben beschrieben synthetisiert. Nach Acetylierung des N-Terminus erfolgt die Spaltung der Dde-Schutzgruppen selektiv durch 3% Hydrazinhydrat.
b) *Farbstoffkopplung*: An die noch festphasengebundenen Peptide werden die Farbstoffe N-terminal bzw. an Lysin gebunden. Hierzu werden 75 µmol des jeweiligen Farbstoffes **1-9 und 13** (1,5 eq) in 600 µl Dimethylformamid gelöst und mit 83 µmol TBTU (2-(1H-Benzotriazol-1-yl)-1,1,3,3-tetramethyluronium-tetrafluoroborat) sowie 150 µmol N,N-Diisopropylethylamin versetzt. Nach 2 min wird dieses Reaktionsgemisch auf das jeweilige peptidtragende Harz gegeben und über Nacht reagieren lassen. Anschließend wird das Harz 5x mit Dimethylformamid und 3x mit Dichlormethan gewaschen und an der Luft getrocknet.
c) *Schutzgruppenabspaltung und Ablösung der Farbstoff-Peptid-Konjugate*: Je 1,5 ml einer Mischung bestehend aus 750 mg Phenol, 250 µl Ethandithiol, 500 µl Thioanisol und 500 µl Wasser in 10 ml Trifluoressigsäure werden auf das peptidkonjugattragende Harz gegeben und für vier Stunden einwirken lassen. Die Farbstoff-Peptid-Konjugate werden mit kaltem t-Butylmethylether gefällt, 6x mit kaltem Diethylether gewaschen, in 5%iger Essigsäure gelöst und gefriergetrocknet. Reinheitsanalyse und Reinigung der Konjugate erfolgt mittels RP-HPLC an einer Vydac-C18-Säule (Gradient: Wasser+0,05% TFA /Acetonitril, 5% auf 60% Acetonitril in 20 min, Detektion: 214 und 750 nm).

Die Strukturen der synthetisierten Farbstoff-Peptid-Konjugate sind in der folgenden Übersicht zusammengefaßt:

### Farbstoffkonjugate mit VIP-Rezeptor-bindenden Peptiden I

### Beispiel 14 bis 16: Konjugate von Farbstoff 1-3 mit VIP(1-28)

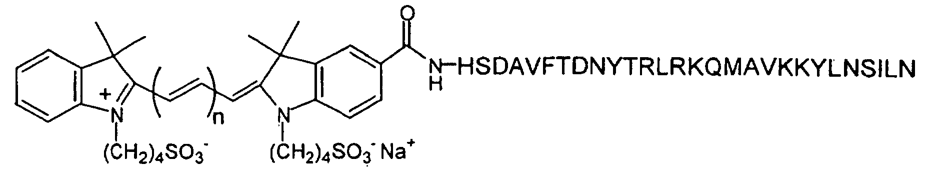

n = 1: Indocarbocyanin-VIP(1-28)-Konjugat **14**
n = 2: Indodicarbocyanin-VIP(1-28)-Konjugat **15**
n = 3: Indotricarbocyanin-VIP(1-28)-Konjugat **16**

### Beispiel 17: Konjugat von Farbstoff 12 mit Cys¹⁷-VIP(1-28)

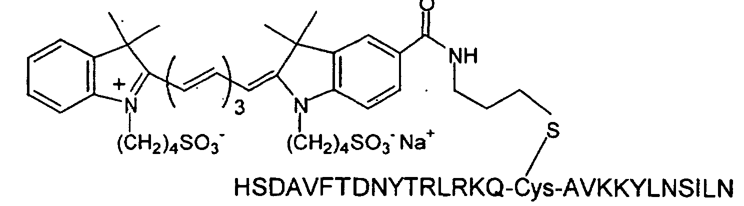

Indotricarbocyanin-Cys¹⁷-VIP(1-28)-Konjugat **17**

### Beispiel 18 bis 20: Konjugate von Farbstoff 1-3 mit VIP(14-28)

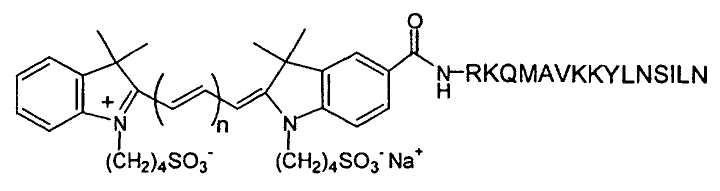

n = 1: Indocarbocyanin-VIP(14-28)-Konjugat **18**
n = 2: Indodicarbocyanin-VIP(14-28)-Konjugat **19**
n = 3: Indotricarbocyanin-VIP(14-28)-Konjugat **20**

Beispiel 21 bis 23: Konjugate von Farbstoff **4-6** mit VIP(14-24) 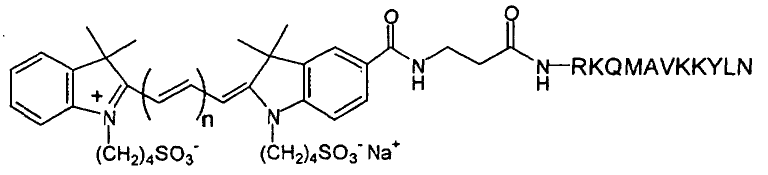 n = 1: Indocarbocyanin-β-Alanin-VIP(14-24)-Konjugat **21**
n = 2: Indodicarbocyanin-β-Alanin-VIP(14-24)-Konjugat **22**
n = 3: Indotricarbocyanin-β-Alanin-VIP(14-24)-Konjugat **23**

### Beipiel 17: Harzsynthese von Peptidkonjugaten bestehend aus VIP-rezeptorbindenden Peptiden und den Farbstoffen 10-12.

Um die Bromtragenden Farbstoffe **10-12** chemoselektiv über eine Thioetherbindung an die Peptide zu binden, muß in das Peptid ein Cystein oder Homocystein mit orthogonaler Schutzgruppe eingebaut werden. Die Festphasenpeptidsynthese wird wie für die Beispiele 14-16/18-27 beschrieben durchgeführt und der Baustein Fmoc-cys(Mmt)-OH eingesetzt. Die Monomethoxytritylgruppe (Mmt) ist unter Erhalt der übrigen Seitenschutzgruppen durch 1% TFA/5% Triisobutylsilan in Dichlormethan abspaltbar. Hierzu wird das Harz 3 mal mit je 1ml obiger Lösung für 10 min inkubiert. Nach Waschen des Harzes mit Dichlormethan (3x), DMF (5x) und Ethanol (3x) wird das Harz für 2 min mit einer 20%igen Cäsiumcarbonatlösung (1 ml) inkubiert und anschließend mit Wasser (2x), Ethanol (2x) und DMF (2x) gewaschen. Anschließend werden 75 µmol des jeweiligen Farbstoffes **10-12** (1,5 eq) in 600 µl Dimethylformamid gelöst und auf das jeweilige Harz gegeben und der Vorgang nach 30 min wiederholt. Anschließend wird das Harz fünf mal mit Dimethylformamid (5x) und mit Dichlormethan (2x) gewaschen. Nach Trocknung des Harzes an Luft erfolgt die Schutzgruppenabspaltung und Ablösung vom Träger wie oben beschrieben.

Die Strukturen von weiteren synthetisierten Farbstoff-Peptid-Konjugaten sind in der folgenden Übersicht zusammengefaßt:

### Farbstoffkonjugate mit VIP-Rezeptor-bindenden Peptiden II

### Beispiel 24: Konjugat von Farbstoff 6 mit Lys²⁵-VIP(14-25)

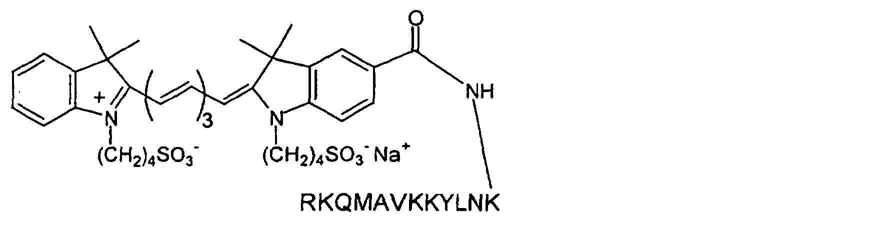

(ε-Amino-Indotricarbocyanin)-Lys²⁵-VIP(14-25)-Konjugat **24**

### Beispiel 25: Konjugat von Farbstoff 3 mit D-VIP(14-24)

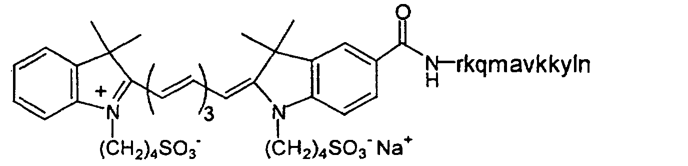

Indotricarbocyanin-D-VIP(14-24)-Konjugat **25**

### Beispiel 26: Konjugat von Farbstoff 13 mit D-VIP(14-24)

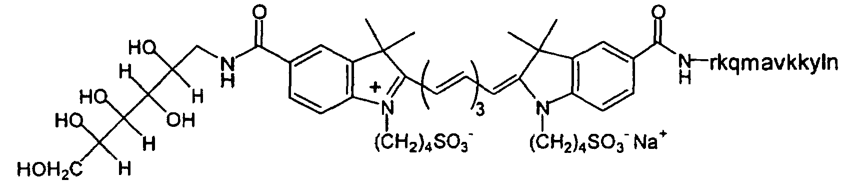

Indotricarbocyanin-5-carbonsäureglucamid-D-VIP(14-24)-Konjugat **26**

### Beispiel 27: Konjugat von Farbstoff 13 mit retro-D-VIP(14-24)

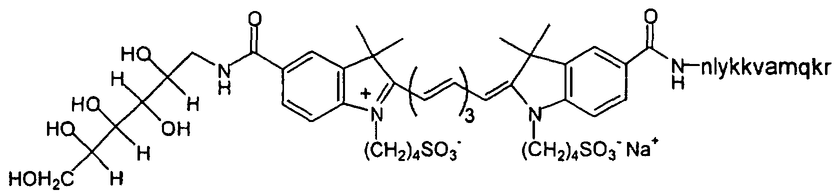

Indotricarbocyanin-5-carbonsäureglucamid-*retro*-D-VIP(14-24)-Konjugat **27**

### Beipiele 28 bis 32: Harzsynthese von Peptidkonjugaten bestehend aus somatostatinrezeptorbindenden Peptiden und den Farbstoffen 1-9 und 13.

### Allgemeine Vorschrift:

Die Synthesen wurden an 500 mg TCP-Thr(But)-fmoc-Harz (Fa. Pepchem Tübingen) mit einer Beladung von 0,49 mmol/g durchgeführt. Das Peptid wird "step by step" unter Verwendung folgender temporärer Schutzgruppen synthetisiert: tert.-butyl für Thr und Ser, Trityl für Cys und Asp, Boc für Trp und Lys. Als Kondensationsreagenz wird HBTU verwendet.

Nach Abspaltung der N-terminalen Fmoc-Schutzgruppe wird der Farbstoff **1-9, 13** in einem gesonderten Syntheseschritt kondensiert. Dazu wird 255 mg Harz in ca. 2 ml DMF suspendiert und mit 0,5 mmol Farbstoff, 0,5 mmol HBTU und 0,17 ml DIEA versetzt. Es wird 18 h bei Raumtemp. gerührt, das Harz abgesaugt, mit Dichlormethan gewaschen und getrocknet. Die Abspaltung des Farbstoff-Peptidkonjugates vom Harz erfolgte mit 95% Trifluoressigsäure unter Zusatz von Triisopropylsilan mit anschließender Lyophilisierung aus 10% Essigsäure. Das Rohprodukt wird mittels Aktivkohle cyclisiert und chromatographisch gereinigt (50x300mm VYDAC RP-18, Gradient: Wasser/Acetonitril).

Die Strukturen der synthetisierten Farbstoff-Peptid-Konjugate sind in der folgenden Übersicht zusammengefaßt:

### Farbstoffkonjugate mit Somatostatinrezeptorbindenden Peptiden

### Beispiel 28 bis 30: Konjugat von Farbstoff 1-3 mit Pentetreotid

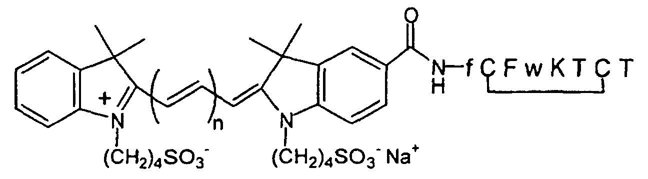

n = 1: Indocarbocyanin-pentetreotid **28**
n = 2: Indodicarbocyanin-pentetreotid **29**
n = 3: Indotricarbocyanin-pentetreotid **30**

### Beispiel 31: Konjugat von Farbstoff 3 mit Somatostatin-14

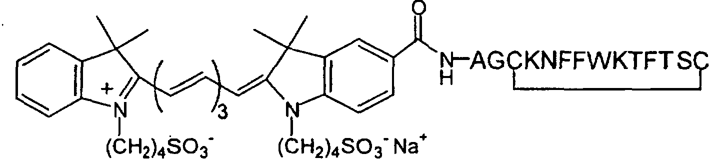

Indotricarbocyanin-Somatostatin-14-Konjugat **31**

### Beispiel 32: Konjugat von Farbstoff 13 mit Somatostatin-14

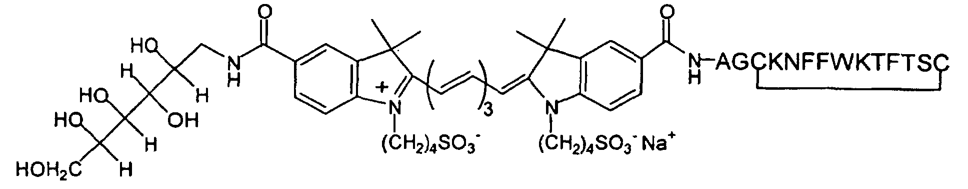

Indotricarbocyanin-5-carbonsäureglucamid-Somatostatin-14-Konjugat **32**

### Beipiele 33 bis 38: Synthese von Peptidkonjugaten aus Neurotensin-Peptiden und dem Farbstoff 3.

Die Synthese der Substanzen erfolgt analog den für die Beispiele 14-27 beschriebenen, allgemeinen Protokollen.

Die Strukturen der synthetisierten Farbstoff-Peptidkonjugate sind in der folgenden Übersicht zusammengefaßt:

### Farbstoffkonjugate mit Neurotensinrezeptorbindenden Peptiden

Beispiel 33 bis 35: Konjugat von Farbstoff **7-9** mit D-Tyr¹¹-Neurotensin(7-13) 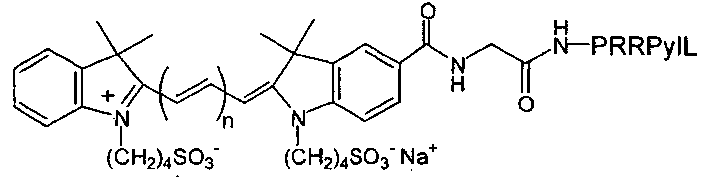 n = 1: Indocarbocyanin-D-Tyr¹¹-Neurotensin(7-13)-Konjugat **33**
n = 2: Indodicarbocyanin-D-Tyr¹¹-Neurotensin(7-13)-Konjugat **34**
n = 3: Indotricarbocyanin-D-Tyr¹¹-Neurotensin(7-13)-Konjugat **35**

### Beispiel 36: Konjugat von Farbstoff 2 mit D-Tyr¹¹-Neurotensin

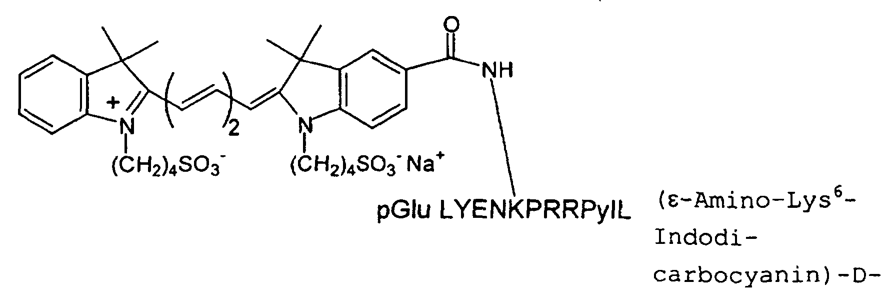

### Beispiel 37 bi 38: Konjugat von Farbstoff 10-11 mit D-Tyr¹¹-Neuro-tensin(5-13)-Cys

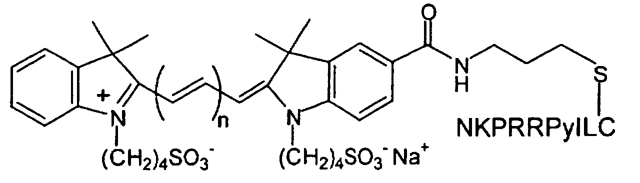

n = 1: Indocarbocyanin-D-Tyr¹¹-Neurotensin(5-13)-Cys-Konjugat **37**
n = 2: Indodicarbocyanin-D-Tyr¹¹-Neurotensin(5-13)-Cys-Konjugat **38.**

### Beispiel 39: Absorptions- und Fluoreszenzeigenschaften der synthetisierten Farbstoff-Peptidkonjugate

Absorptionsmaxima und Extinktionskoeffizienten wurden in PBS und in Rinderplasma bestimmt (Perkin Elmer Lambda 2). Fluoreszenzemissionsspektren wurden in PBS durch Anregung auf der kurzwelligen Seite (ca. 40 nm vom Absorptionsmaximum) erhalten (SPEX Fluorolog, R928 PMT).

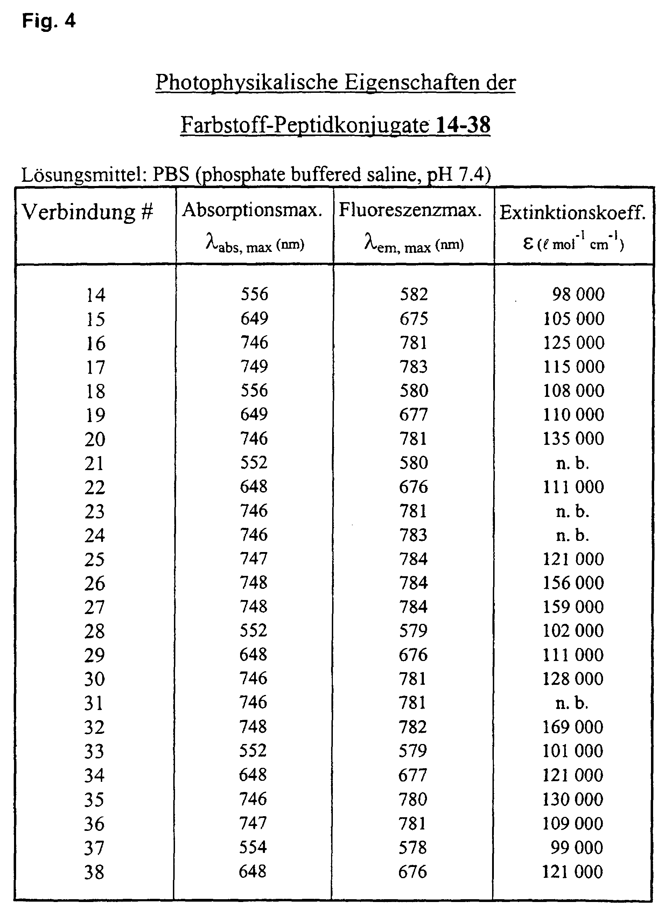

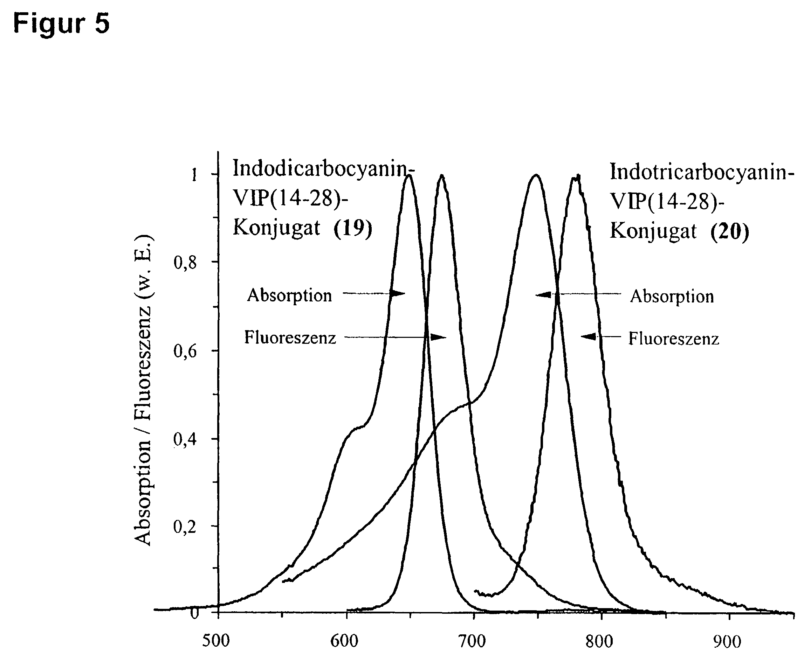

Die Absorptions- und Fluoreszenzdaten sind in Fig. 4 zusammengefaßt. In Fig. 5 sind typische Absorptions- und Fluoreszenzemissionsspektren beispielhaft gezeigt.

### Beispiel 40: Bestimmung der Zellaufnahme mittels Fluoreszenzmikroskopie

Die Bindung und Aufnahme der erfindungsgemäßen Verbindungen wurden in vitro an humanen Tumorzellen untersucht, die Rezeptoren für vasoaktives intestinales Peptid und/oder Somatostatin und/oder Neurotensin exprimieren. Dazu wurden 5 x 10⁵ Tumorzellen in 1,5 ml Medium inkubiert, welches die Testsubstanz enthielt. Es wurden unterschiedliche Konzentrationen der Substanzen (10 nM-10 µM) eingesetzt und die Inkubationsdauer variiert (1 min-24 h). Nach der Inkubation wurden die Zellen fixiert und mikroskopische Präparate hergestellt. Die Auswertung erfolgte an einem Zeiss Axiovert 135-Fluoreszenzmikroskop, das mit einem Cy 7- (Exciter HQ 710/70nm, Emitter 810/90nm, Beamsplitter 750nm LP), Cy5- (Exciter 575-625 nm, Emitter 660-710nm BP, Beamsplitter 645nm) und Cy3-Filtersatz (Exciter 546/12nm, Emitter 590nm LP, Beamsplitter 580nm) ausgestattet war. Von allen Präparaten wurden Weißlicht- und Fluoreszenzbilder mit einer CCD-Kamera (Visitron RTE/CCD-576) aufgenommen und digital gespeichert.

### Ausgwählte Ergebnisse sind im folgenden beschrieben:

Es wurden mikroskopische Weisslicht- und Fluoreszenzaufnahmen (Cy7-Filtersatz) von HT29-Zellen nach 30 min Inkubation mit 10 µM Indotricarbocyanin-VIP(1-28)-Konjugat **16** erstellt. Im Fluoreszenzbild wurde ein weitgehend homogen über die Zelle verteilte Fluoreszenz detektiert. Zusätzlich wurden Areale erhöhter Signale sichtbar, die mit vesikulären Kompartimenten assoziiert sein können. Die Zellen im Fluoreszenzbild korrelieren in ihrer räumlichen Ausdehnung mit dem Weisslichtbild.

Mit folgenden Verbindungen wurden Weisslicht- und Fluoreszenzaufnahmen (Cy7-Filtersatz) in analoger Durchführung erhalten:
Indotricarbocyanin-VIP(14-28)-Konjugat **20,** 10 µM, HT29-Zellen. Homogen über die Zelle verteilte Fluoreszenz, gute Korrelation mit dem Weisslichtbild.
Indotricarbocyanin-*retro*-D-VIP(24-14) **25,** 10 µM, HT29-Zellen. Homogen über die Zelle verteilte Fluoreszenz, gute Korrelation mit dem Weisslichtbild.
Indotricarbocyanin-pentetreotid-Konjugat **30,** 10 µM, RIN38-VIP1-Zellen. Fluoreszenzareale mit vesikulärem Muster im Membranbereich der Zelle, gute Korrelation mit dem Weisslichtbild.

Des weiteren wurden Fluoreszenzaufnahmen in analoger Durchführung mit den folgenden Verbindungen erhalten: (ε-Amino-Indotricarbocyanin)-Lys²⁵-VIP(14-25)-Konjugat **24,** 10 µM, RIN38-VIP1-Zellen, Cy7-Filtersatz. Das Fluoreszenzbild zeigt eine homogene, intrazelluläre zellkernnahe Fluoreszenz.
Indodicarbocyanin-VIP(1-28)-Konjugat **15,** 10 µM, RIN38-VIP1-Zellen, Cy5-Filtersatz. Das Fluoreszenzbild zeigt intrazelluläre Fluoreszenzareale mit vesikulärem Muster im Membranbereich der Zelle.

Indocarbocyanin-VIP(1-28)-Konjugat **14,** 10 µM, RIN38-VIP1-Zellen, Cy3-Filtersatz. Das Fluoreszenzbild zeigt intrazelluläre Fluoreszenzareale mit vesikulärem Muster im Membranbereich der Zelle.

### Beispiel 41: Untersuchung der Tumoranreicherung mittels In-vivo-Fluoreszenzbildgebung an tumortragenden Mäusen

Die bildgebenden Eigenschaften der erfindungsgemäßen Verbindungen wurden in vivo nach Injektion in tumortragende Nacktmäuse untersucht. Dazu wurden 0,1 µmol/kg bis 2 µmol/kg der Substanz intravenös appliziert und die Anreicherung in der Tumorregion in einem Zeitraum von 0 bis 48 Stunden beobachtet. Die Fluoreszenz der Substanzen wurde durch Bestrahlung der Tiere mit Nahinfrarot-Licht der Wellenlänge 640 nm (Indodicarbocyanine) bzw.740 nm (Indotricarbocyanine), das mit einem Nd:YAG Laser erzeugt wurde, angeregt. Die Fluoreszenzstrahlung wurde bei einer Wellenlänge von >700nm bzw. > 800 nm durch eine intensivierte CCD-Kamera detektiert und die Fluoreszenzbilder digital gespeichert.

### Ausgewählte Ergebnisse sind im folgenden beschrieben:

Von einer tumortragenden Nacktmaus (HT29-Tumor in der rechten Hinterflanke) wurden Ganzkörper-Fluoreszenzbilder vor und 1 h nach Applikation von 0,1 µmol/kg Indotricarbocyanin-VIP(14-28)-Konjugat **20** aufgenommen. Die Fluoreszenzintensität vor Applikation ist vernachlässigbar (geringe Autofluoreszenz). 1 h nach Applikation resultiert ein ca. 2-fach erhöhtes Signal im Tumor relativ zur kontralateralen Flanke bei ansonsten homogen über den Restkörper verteilten Fluoreszenzemission.

Von einer tumortragenden Nacktmaus (RIN38-SSTR2-Tumor in der rechten Hinterflanke) wurden Ganzkörper-Fluoreszenzbilder vor und 1 h nach Applikation von 0,1 µmol/kg Indotricarbocyanin-pentetreotid-Konjugat **30** aufgenommen. Die Fluoreszenzintensität vor Applikation ist vernachlässigbar (geringe Autofluoreszenz). 1 h nach Applikation resultiert ein ca. 3-fach erhöhtes Signal im Tumor relativ zur kontralateralen Flanke bei ansonsten homogen über den Restkörper verteilten Fluoreszenzemission.

Von einer tumortragenden Nacktmaus (HT29-Tumor in der rechten Hinterflanke) wurden Ganzkörper-Fluoreszenzbilder vor und 1 h nach Applikation von 0,1 µmol/kg (ε-Amino-Indotricarbocyanin)-Lys²⁵-VIP(14-25)-Konjugat **24** aufgenommen. Die Fluoreszenzintensität vor Applikation ist vernachlässigbar (geringe Autofluoreszenz). 1 h nach Applikation resultiert ein ca. 1,5-fach erhöhtes Signal im Tumor relativ zur kontralateralen Flanke. Zusätzlich wurde ein erhöhtes Fluoreszenzsignal in den Nieren detektiert.

Von einer tumortragenden Nacktmaus (HT29-Tumor in der rechten Hinterflanke) wurden Ganzkörper-Fluoreszenzbilder vor und 5 min nach Applikation von 0,2 µmol/kg Indodicarbocyanin-VIP(1-28)-Konjugat **15** aufgenommen. Die Fluoreszenzintensität vor Applikation ist vernachlässigbar (geringe Autofluoreszenz). 1 h nach Applikation resultiert ein ca. 1,4-fach erhöhtes Signal im Tumor relativ zur kontralateralen Flanke. Zusätzlich wurde ein erhöhtes Fluoreszenzsignal in den Nieren detektiert.

### Beispiel 42: Untersuchung der Stabilität von Farbstoff-Peptidkonjugaten in Rinderplasma

Die chemische Stabilität der erfindungsgemäßen Verbindungen in Plasma wurde in vitro in Abhängikeit von der Zeit mittels HPLC untersucht. Dazu wurden 1 mM Lösungen der Peptide in PBS in Rinderblutplasma (Fa. Graeber, gefroren, zur Heparinanalyse) unter Erhalt einer Konzentration von 30 µM pipettiert und die Lösungen bei 37 °C inkubiert.
Zu verschiedenen Zeitpunkten (0,5; 1; 2; 4; 6; 24 h) erfolgte die Aufarbeitung der Proben, indem 1 ml der Plasmalösung mit 1 ml MeOH versetzt wird und die ausgefallenen Proteine abzentrifugiert werden.
Die Analyse des Überstandes erfolgte mittels HPLC durch Bestimmung des Gehaltes bei' 750 nm bezogen auf den Gehalt nach 1 min Inkubation bei 0 °C (Kontrolle).
HPLC: Beckmann, Diodenarraydetektor TIDAS (Fa. J&M) 350-1000 nm;
Säule: Chromasil 5µ, 250mm x 4,5mm
Laufmittel: A: 90% H₂O(+0.5%TFA)/10% MeOH
   B: 10% H₂O(+0.5%TFA)/90% MeOH
Gradient: 10% B auf 100% B innerhalb von 20 min

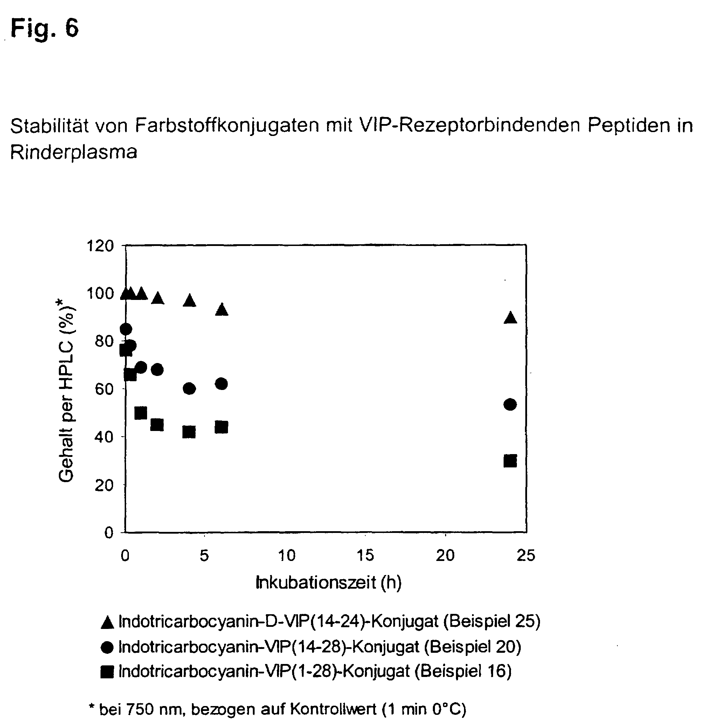

Die Beispiele sind in Fig. 6 zusammengefaßt.

### Beispiel 43: Substitutionsanalyse von VIP mittels Spotsynthese

### 1. Synthese von Peptiden auf Cellulose

Die Synthese von Peptiden auf Zellulose (Spotsynthese) wurde 1988 erstmals von R. Frank und R. Döring veröffentlicht und 1992 detailliert von R. Frank¹ beschrieben. Hier wurde die in der AG Schneider-Mergener etablierte Methode² angewendet.

Die Zellulosemembran wurde chemisch modifiziert, um geeignete Ankerfunktionen für die nachfolgende Peptidsynthese anzubringen. Dabei wurde auf eine aminofunktionalisierte Zellulose-Membran (CAPE-Membran)³ eine Mercaptofunktion⁴ eingeführt. An diese Mercaptofunktion konnte die erste Aminosäure in Form eines Brompropylesters gekoppelt werden. Anschließend wurden alle Aminosäuren des Peptids sukzessive nach der Fmoc-Strategie aufgebaut. Am Ende wurde der Indodicarboyaninfarbstoff N-terminal an das Peptid gebunden und anschließend alle Seitenschutzgruppen abgespalten.
Um die Peptide auf Rezeptorbindung untersuchen zu können, mußten die Peptide von der Zellulose abgespalten werden. Dafür wurde eine Methode entwickelt, bei der es erstmalig gelungen ist, Peptide mit authentischen C-Terminus von der Zellulose abzuspalten.
1a. Modifikation der Zellulose-Membran
   - Eine 20 x 30 cm große Zellulose-Membran (Whatman 50) wurde für 2 min. mit Methanol/1.2 % Perchloressigsäure inkubiert und anschließend getrocknet.
   - Nach dreistündiger Inkubation mit 10%Epibromhydrin in Dioxan/1,2 % Perchloressigsäure wurde für 30 min. mit Methanol abreagiert und danach zweimal mit Methanol gewaschen.
   - Anschließend wurde 3 x mit Dimethylformamid (DMF) gewaschen und über Nacht mit 50 % 1,3-Diaminopropan (v/v) in DMF inkubiert. Danach wurde wie folgt gewaschen: 3 x DMF, 2 x Ethanol, 2 x Aqua dest., 2 x Ethanol, 15 min. mit 5 M Natriummethanolat, 3 x Methanol, 4 x Aqua dest., 3 x Ethanol, 1 x Diethylether
1b. Definition der Spots
   - Für die Spotdefinition wurde 1.3 µl 0.6 M Fmoc-β-Alanin-Opfp-Lösung mit dem Auto-Spot Robot 222 XL (Abimed, Langenfeld) bei einer Reaktionszeit von 15 min. auf bestimmte Punkte der Zellulose-Membran doppelt aufpipettiert.
   - Die Membran wurde 2 min. mit 2 % Acetanhydridlösung und 30 min. mit 20 % Acetanhydrid/10 % Diisopropylethylamin acetyliert.
   - Für die Abspaltung der Fmoc-Schutzgruppe wurde die Membran 3 x mit DMF gewaschen, 2 x 10 min. mit 2:0 % Piperidin-Lösung inkubiert, 5 x mit DMF und 1 x mit Ethanol gewaschen. Die freien Aminogruppen konnten mit Bromphenolyblau sichtbar gemacht werden. Nach wiederholtem Waschen mit Ethanol wurde die Membran getrocknet.
1c. Kopplung der Mmt-Mercaptopropionsäure und des Brompropylesters
   - Es wurde 0.6 M Mercaptopropionsäure bei einer Reaktionszeit von 15 min. doppelt auf die definierten Spots pipettiert. Anschließend wurde 3 x mit DMF und 3 x mit Dichlormethan (DCM) gewaschen..
   - Die Abspaltung der Mmt-Schutzgruppe erfolgte mit Inkubation für 2 min. mit 10 % Dichloressigsäure/0.5% Trifluoressigsäure und 3 x 5 min. mit 10 % Dichloressisäure/0,5 % trifluoressigsäure/5 % Triisobutylsilan. Folgende Waschschritte wurden durchgeführt: 1x DCM, 2 x Ethanol, 1 x Aqua dest., 1-2 min. mit 10 % Cäsiumcarbonat, 1 x Aqua dest., 2 x Ethanol, 1 x Diethylether
   - Die jeweiligen Fmoc-brompropyl-aminosäureester wurden 3 x bei einer Konzentration von 0.6 M und Reaktionszeit von 15 min. gekoppelt. Die Abspaltung der Fmoc-Schutzgruppe wurde wie bei 1b durchgeführt.
1d. Kopplung der Aminosäuren
   - Die Peptide wurden durch wiederholtes Aufpipettieren von 0.6 M Aminosäurelösungen in N-Methylpyrrolidon auf die Spots und anschließender Abspaltung der Fmoc-Schutzgruppen aufgebaut.
1e. Kopplung des Indodicarbocyaninfarbstoffes
   - Es wurde eine 0.3 M Lösung des Indodicarcocyaninfarbstoffes mit 0.3 M TBTU und 0.6 M Diisopropylethylamin aktiviert und 4 x bei einer Reaktionszeit von 15 min. auf die Spots aufpipettiert.
1f. Abspaltung der Seitenschutzgruppen
   - Die Abspaltung der Seitenschutzgruppen erfolgte durch nacheinanderfolgende Behandlung der Membran mit 90 % Trifluoressigsäure/3 % Triisobutylsilan/2 % Aqua dest./1 % Phenol für 30 min. und mit 50 % Trifluoressigsäure/3 % Triisobutylsilan/2 % Aqua dest./1 % Phenol für 2,5 h. Anschließend wurde 4 x mit Dichlormethan, 3 x mit DMF und 1 x mit Ethanol gewaschen.

### 2. Abspaltung der Peptide von der Zellulose-Membran

- Die Spots wurden ausgestanzt und mit Methanol gewaschen. Für die Abspaltung wurde 30 min. mit 70 mM Natriummethanolat in Methanol inkubiert. Der pH konnte durch Zugabe von 37%iger Salzsäure korrigiert werden. Anschließend wurden die Peptide in einer Speed-Vac getrocknet.
- Nach dem Trocknen wurden die Peptide in Aqua dest. aufgenommen und mittels Reversed-Phase-HPLC und MALDI-TOF analysiert.

### 3. Zellassay und Durchflußzytometrie

- Die Konzentration der VIP-Derivate wurde photometrisch über den Farbstoff ermittelt. In dem Zellassay wurden die Peptide mit einer Endkonzentration von 150 mM eingesetzt. Dabei wurden 1 x 10⁵ RIN38(VAPC1)-Zellen mit den VIP-Derivaten für eine Stunde bei 37° C in Bindungspuffer (50 mM Tris/HCL, pH 7,5 , 5 nM MgCl₂, 1 mM CaCl₂, 100 mM NaCl, 4 % BSA) inkubiert.
- Anschließend wurden die Zellen 2 x mit PBS gewaschen, in FACS-Röhrchen überführt und 5 min. bei 377 g zentrifugiert. Das Zellpellet wurde in 300 µl Cellfix resuspendiert am FACS-Calibur (Becton Dickinson) mit FL4-Optik gemessen.

### 4. Auswertung

- Die Fluoreszenzintensitäten der in der Durchflußzytometrie gemessenen nativen, nätürlich vorkommenden, humanen VIP-Peptide wurde 100 % gesetzt. Die Standardabweichung von 28 nativen VIP-peptiden betrug 11%. Die weiteren VIP-Derivate wurden diesen 100 % angeglichen.

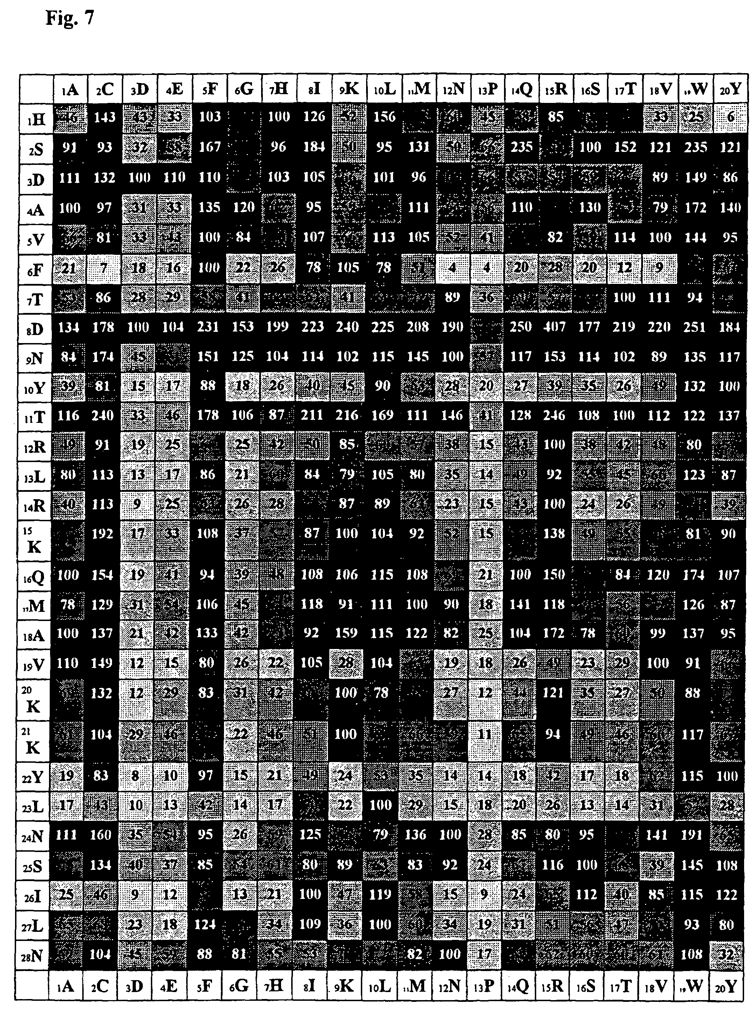

Fig. 7 zeigt relative Fluoreszenzintensitäten von RIN38 VPAC1-Zellen nach Inkubation in Gegenwart von 150 nM der farbstoffmarkierten Peptide für 1 h bei 37 °C. Angaben in Prozent bezogen auf das native Peptid der jeweiligen Reihe.

### Literatur zur Spotsynthese:

1. Frank, R. (1992) Spot synthesis: an easy technique for the positionally addressable, parallel chemicäl synthesis on a membrane support. *Tetrahedron* 48, 9217-9232
2. Kramer, A., Schneider-Mergener, J. (1998) Synthesis and screening of peptide libraries on continuos cellulose membrane supports. *Methods in Molecular Biology* 87, 25-39
3. Volkmer-Engert, R., Hoffmann, B., Schneider-Mergener, J. (1997) *Tetrahedron Lett*. 38, 1029-1032
4. Licha, K., Bhargava, S., Rheinländer, C., Becker, A., Schneider-Mergener, J., Volkmer-Engert, R. (in press) Highly paralles Nano-synthesis of cleavable peptidedye conjugates on cellulose membranes. *Tetrahedron Lett*.

### Beispiel 44

### a) 5-N-(2,3-Dihydroxypropyl)aminocarbonyl-1-(4-sulfobutyl)-2,3,3-trimethyl(3H)indolenin

0,9 g (2,6 mmol) 5-Carboxy-1-(4-sulfobutyl)-2,3,3-Trimethyl(3H)indolenin (Anal. Biochem. 217,197, 1994) werden in 30 ml absolutem N,N-Dimethylformamid und 3 ml Pyridin vorgelegt und mit 1,35 g (5,3 mmol) Disuccinimidylcarbonat versetzt. Nach drei Stunden addiert man 0,965 g (10,6 mmol) 2,3-Dihydroxypropylamin. Man rührt über Nacht bei Raumtemperatur, dampft den Ansatz zur Trockne ein und rührt den Rückstand mit Diethylether aus. Der Feststoff wird abgesaugt und zur Reinigung an RP-Material chromatographiert. Ausbeute: 0,82 g (76 % d. Th.)

| Analyse (bezogen auf lösungsmittelfreie Substanz): | | | | | |
|---|---|---|---|---|---|
| ber. | C 55,32 | H 6,84 | N 6,79 | S 7,77 | O 23,27 |
| gef. | C 55,39 | H 6,95 | N 6,57 | S 7,58 | |

### b) 4-[2-[4-Chlor-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-. (dihydroxypropyl)aminocarbonyl-3,3-dimethyl(3H)indolio]butansulfonat, Natriumsalz

Eine Lösung von 360 mg (1 mmol) N-[5-Anilino-3-chlor-2,4-(propan-1,3-diyl)-2,4-pentadien-1-yliden)aniliniumchlorid, 825 mg (2 mmol) 5-N-(2,3-Dihydroxypropyl)aminocarbonyl-1-(4-sulfobutyl)-2,3,3-trimethyl(3H)indolenin (Beispiel 44a) und 330 mg (4 mmol) wasserfreies Natriumacetat in 30 ml Ethanol werden für zwei Stunden unter Argon am Rückfluß gekocht. Anschließend destilliert man das Ethanol ab und reinigt den Rückstand chromatographisch.
Ausbeute: 0,58 g (59 % d. Th.)

| Analyse (bezogen auf lösungsmittelfreie Substanz): | | | | | | | |
|---|---|---|---|---|---|---|---|
| ber. | C 56,17 | H 6,15 | Cl 3,60 | N 6,79 | S 7,77 | 0 23,27 | Na 2,34 |
| gef. | C 55,99 | H 6,30 | Cl 3,41 | N 6,87 | S 7,64 | | Na 2,17 |

### c) 4-[2-[4-(4-(2-Carboxyethyl)phenyloxy)-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)aminocarbonyl-3,3-dimethyl(3H)indolio]butansulfonat, Natriumsalz, N-Hydroxysuccinimidester

225 mg (1,4 mmol) 3-(4-Hydroxyphenyl)propionsäure werden in 10 ml trockenem N,N-Dimethylformamid unter Schutzgas gelöst und mit 65 mg (2,7 mmol) Natriumhydrid (60%-ig in Öl) versetzt. Nach 30 Minuten addiert man 138 mg (0,14 mmol) 4-[2-[4-Chlor-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)aminocarbonyl-3,3-dimethyl(3H)indolio]butansulfonat, Natriumsalz (Beispiel 44b) und rührt für weitere 30 Minuten. Anschließend wird die Reaktionsmischung mit Trockeneis gequencht und zur Trockne eingedampft. Der Rückstand wird über eine präparative HPLC gereinigt. Zur Herstellung des Aktivesters löst man 14 mg (120 µmol) N-Hydroxysuccinimid und 2 mg (2,4 µmol) der Carbonsäure in 200 µl N,N-Dimethylformamid. Nach zehn Minuten setzt man 24 mg (120 µmol) Dicyclohexylcarbodiimid zu und rührt über Nacht bei Raumtemperatur. Der Aktivester wird ohne weitere Reinigung in die nächste Stufe eingesetzt.

Analog zur Synthese nach Beispiel 14-16 und 18-27 a (Festphasenpeptidsynthese) wird das VIP-Analogon HSDAVFWDNY TRLRKQMAVK KYLNSILN an der Festphase synthetisiert. Der Farbstoff 4-[2-[4-(4-(2-Carboxyethyl)-phenyloxy)-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)aminocarbonyl-3,3-dimethyl(3H)indolio]-butansulfonat, Natriumsalz wird nach Beispiel 14-16 und 18-27 b (Farbstoffkopplung) an das Peptid gekoppelt und nach Beispiel 14-16 und 18-27 c (Schutzgruppenabspaltung und Ablösung der Farbstoff-Peptid-Konjugate) wird dieses Konjugat isoliert und gereinigt.

### d) 4-[2-[4-(4-(2-Isothiocyanatoethyl)phenyloxy)-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)aminocarbonyl-3,3-dimethyl(3H)indolio]butansulfonat, Natriumsalz

Zu einer Suspension von 28 mg (0,6 mmol) Natriumhydrid (60%-ig in Öl) in 4 ml wasserfreiem N,N-Dimethylformamid addiert man bei 0°C 116 mg (0,6 mmol) 2-(4-Hydroxyphenyl)ethylisothiocyanat in 2 ml N,N-Dimethylformamid. Nach 30 Minuten wird die so hergestellte Lösung zu 138 mg (0,14 mmol) 4-[2-[4-Chlor-7-[5-N-(dihydroxypropyl)aminocarbonyl-5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-3,3-dimethyl(3H)indolio]butansulfonat, Natriumsalz (Beispiel 44b) gegeben. Man rührt über Nacht bei Raumtemperatur und quencht anschließend mit Trockeneis. Man dampft am Rotationsverdampfer zur Trockne ein und reinigt den Rückstand per präparativer HPLC.
Ausbeute: 85 mg (54 % d. Th.)

| Analyse (bezogen auf lösungsmittelfreie Substanz): | | | | | | |
|---|---|---|---|---|---|---|
| ber. | C 58,65 | H 6,09 | N 6,22 | S 8,54 | 0 18,47 | Na 2,04 |
| gef. | C 58,53 | H 6,17 | N 6,11 | S 8,63 | | Na 1,83 |

Analog zur Synthese nach Beispiel 14-16 und 18-27 a (Festphasenpeptidsynthese) wird das VIP-Analogon HSDAVFTDNY TRLRFQMAVK KYLNSILN an der Festphase synthetisiert. Der Farbstoff 4-[2-[4-(4-(2-Isothiocyanatoethyl)-phenyloxy)-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]- 5-N-(dihydroxypropyl)aminocarbonyl-3,3-dimethyl(3H)indolio]-butansulfonat, Natriumsalz wird an das Peptid N-terminal gekoppelt und nach Beispiel 14-16 und 18-27 c (Schutzgruppenabspaltung und Ablösung der Farbstoff-Peptid-Konjugate) wird dieses Konjugat isoliert und gereinigt.

In analoger Weise können weitere symmetrische hydrophile Farbstoffe aus folgenden Bausteinen aufgebaut werden:

### Hydrophile Indolenin-Derivate mit Hydroxyalkylsubstituenten: (hergestellt nach Beispiel 44a)

a) 5-N-(2,3-Dihydroxypropyl)-N-methylaminocarbonyl-1-(4-sulfobutyl)-2,3,3-trimethyl(3H)indolenin
b) 5-N-(Hydroxyethyl)-aminocarbonyl-1-(4-sulfobutyl)-2,3,3-trimethyl(3H)indolenin
c) 5-N-(2,3-Dihydroxypropyl)-N-(hydroxyethyl)-aminocarbonyl-1-(4-sulfobutyl)-2,3,3-trimethyl(3H)indolenin
d) 5-N,N-(bis-Hydroxyethyl)-aminocarbonyl-1-(4-sulfobutyl)-2,3,3-trimethyl(3H)indolenin
e) 5-N-(2,3,4,5,6-Pentahydroxyhexyl)-aminocarbonyl-1-(4-sulfobutyl)-2,3,3-trimethyl(3H)indolenin
f) 5-N-(1,3,4-Trihydroxybut-2-yl)-N-methylaminocarbonyl-1-(4-sulfobutyl)-2,3,3-trimethyl(3H)indolenin

### Beispiel 45

### a) 5-N-(2,3,4,5,6-Pentahydroxyhexyl)aminocarbonyl-1-(4-sulfobutyl)-2,3,3-trimethyl(3H)indolenin

0,6 g (1,8 mmol) 5-Carboxy-1-(4-sulfobutyl)-2,3,3-Trimethyl(3H)indolenin (Anal. Biochem. 217, 197, 1994) werden in 20 ml absolutem N,N-Dimethylformamid und 2 ml Pyridin vorgelegt und mit 0,95 g (3,6 mmol) Disuccinimidylcarbonat versetzt. Nach zwei Stunden addiert man 1,4 ml (10 mmol) Triethylamin und 322 mg (1,8 mmol) Glucamin. Man rührt über Nacht bei Raumtemperatur, dampft den Ansatz zur Trockne ein und rührt den Rückstand mit Diethylether aus. Der Feststoff wird abgesaugt und zur Reinigung an RP-Material chromatographiert.
Ausbeute: 0,67 g (74 % d. Th.)

| Analyse (bezogen auf lösungsmittelfreie Substanz): | | | | | |
|---|---|---|---|---|---|
| ber. | C 52,58 | H 6,82 | N 5,57 | S 6,38 | O 28,65 |
| gef. | C 52,47 | H 6,91 | N 5,39 | S 6,44 | |

### b) 4-[2-[4-Chlor-7-[5-N-(2,3,4,5,6-pentahydroxyhexyl)aminocarbonyl-3,3-dimethyl-1-(4-sulfonatobutyl)indolin-2-yliden]-3,5-(propan-1,3-diyl)-1,3,5-heptatrien-1-yl]-3,3-dimethyl5-[N-(2,3,4,5,6-pentahydroxyhexyl)aminocarbonyl] (3H)indolio]butansulfonat, Natriumsalz

Eine Lösung von 180 mg (0,5 mmol) N-[5-Anilino-3-chlor-2,4-(propan-1,3-diyl)-2,4-pentadien-1-yliden]aniliniumchlorid, 503 mg (1 mmol) 5-N-(2,3,4,5,6-Pentahydroxyhexyl)aminocarbonyl-1-(4-sulfobutyl)-2,3,3-trimethyl(3H)indolenin (Beispiel 45a) und 165 mg (2 mmol) wasserfreies Natriumacetat in 10 ml Ethanol werden für zwei Stunden unter Argon am Rückfluß gekocht. Anschließend destilliert man das Ethanol ab und reinigt den Rückstand chromatographisch.
Ausbeute: 0,31 g (53 % d. Th.)

| Analyse (bezogen auf lösungsmittelfreie Substanz) : | | | | | | | |
|---|---|---|---|---|---|---|---|
| ber. | C 54,05 | H 6,33 | Cl 3,01 | N 4,76 | S 5,44 | O 24,45 | Na 1,95 |
| gef. | C 53,89 | H 6,20 | Cl 2,87 | N 4,83 | S 5,29 | | Na 1,72 |

### c) 4-[2-[4-(4-Isothiocyanatothiophenyloxy)-7-[5-N-(2,3,4,5,6-pentahydroxyhexyl)aminocarbonyl-3,3-dimethyl-1-(4-sulfonatobutyl)indolin-2-yliden]-3,5-(propan-1,3-diyl)-1,3,5-heptatrien-1-yl]-3,3-dimethyl-5-[N-(2,3,4,5,6-pentahydroxyhexyl)aminocarbonyl](3H)indolio]butansulfonat, Natriumsalz

54 mg (0,4 mmol) 4-Aminothiophenol werden in 10 ml absolutem N,N-Dimethylformamid unter Argonatmosphäre gelöst und bei Raumtemperatur mit 165 mg (0,14 mmol) 4-[2-[4-Chlor-7-[5-N-(2,3,4,5,6-pentahydroxyhexyl)aminocarbonyl-3,3-dimethyl-1-(4-sulfonatobutyl)indolin-2-yliden]-3,5-(propan-1,3-diyl)-1,3,5-heptatrien-1-yl]-3,3-dimethyl-5-[N-(2,3,4,5,6-pentahydroxyhexyl)aminocarbonyl](3H)indolio]butansulfonat, Natriumsalz (Beispiel 45b) versetzt. Nach 10 Minuten wird die Reaktionsmischung mit Trockeneis gequencht und 210 mg (1 mmol) Thiocarbonyldiimidazol addiert. Nach 45 Minuten wird der Farbstoff mit Diethylether gefällt und der Feststoff per Zentrifugation isoliert. Zur Reinigung kann an RP-Material chromatographiert werden.
Ausbeute: 78 mg (43 % d. Th.)

| Analyse (bezogen auf lösungsmittelfreie Substanz): | | | | | | |
|---|---|---|---|---|---|---|
| ber. | C 55,07 | H 6,01 | N 5,35 | S 9,80 | O 22,01 | Na 1,76 |
| gef. | C 54,89 | H 6,20 | N 5,24 | S 9,58 | | Na 1,54 |

Analog zur Synthese nach Beispiel 14-16 und 18-27 a (Festphasenpeptidsynthese) wird das VIP-Analogon HSWAVFTDNY TRLRKQMAVK KYLNSILN an der Festphase synthetisiert. Der Farbstoff 4-[2-[4-(4-Isothiocyanatothiohenyloxy)-7-[5-N-(2,3,4,5,6-pentahydroxyhexyl)-aminocarbonyl-3,3-dimethyl-1-(4-sulfonatobutyl)indolin-2-yliden]-3,5-(propan-1,3-diyl)-1,3,5-heptatrien-1-yl]-3,3-dimethyl-5-[N-(2,3,4,5,6-pentahydroxyhexyl)aminocarbonyl](3H)indolio]butansulfonat, Natriumsalz wird an das Peptid N-terminal gekoppelt und nach Beispiel 14-16 und 18-27 c (Schutzgruppenabspaltung und Ablösung der Farbstoff-Peptid-Konjugate) wird dieses Konjugat isoliert und gereinigt.

In analoger Weise können weitere symmetrische hydrophile Farbstoffe aus folgenden Bausteinen aufgebaut werden:

### Hydrophile Indolenin-Derivate mit Hydroxyalkylsubstituenten: (hergestellt nach Beispiel 44a)

a) 5-N-(2,3-Dihydroxypropyl)-N-methylaminocarbonyl-1-(4-sulfobutyl)-2,3,3-trimethyl(3H)indolenin
b) 5-N-(Hydroxyethyl)-aminocarbonyl-1-(4-sulfobutyl)-2,3,3-trimethyl(3H)indolenin
c) 5-N-(2,3-Dihydroxypropyl)-N-(hydroxyethyl)-aminocarbonyl-1-(4-sulfobutyl)-2,3,3-trimethyl(3H)indolenin
d) 5-N,N-(bis-Hydroxyethyl)-aminocarbonyl-1-(4-sulfobutyl)-2,3,3-trimethyl(3H)indolenin
e) 5-N-(2,3,4,5,6-Pentahydroxyhexyl)-aminocarbonyl-1-(4-sulfobutyl)-2,3,3-trimethyl(3H)indolenin
f) 5-N-(1,3,4-Trihydroxybut-2-yl)-N-methylaminocarbonyl-1-(4-sulfobutyl)-2,3,3-trimethyl(3H)indolenin

### Beispiel 46

### 7-[5-N-(2,3-Dihydroxypropyl)aminocarbonyl-3,3-dimethyl-1-(4-sulfonatobutyl)indolin-2-yliden)-1,3,5-heptatrien-1-yl]-3,3-dimethyl-5-carboxy(3H)indolio]butansulfonat, Natriumsalz

Eine Lösung von 2,35 g (5,71 mmol) 5-N-(2,3-Dihydroxypropyl)aminocarbonyl-1-(4-sulfobutyl)-2,3,3-trimethyl(3H)indolenin (Beispiel 44a) und 1,57 g (5,5 mmol) Glutaconaldehyddianilid-hydrochlorid in 25 ml Essigsäureanhydrid werden 30 Minuten bei 120°C gerührt. Anschließend addiert man 2,4 g (7,1 mmol) 5-Carboxy-1-(4-sulfobutyl)-2,3,3-trimethyl(3H)indolenin, 1,71 g Natriumacetat, 22 ml Essigsäureanhydrid und 8,6 ml Essigsäure. Man läßt die Reaktionsmischung eine Stunde bei 120°C rühren, kühlt auf Raumtemperatur ab und fällt das Produkt mit Diethylether. Das Rohprodukt wird über RP-Material chromatographiert.
Ausbeute: 1,9 g (40 % d. Th.)

| Analyse (bezogen auf lösungsmittelfreie Substanz): | | | | | | |
|---|---|---|---|---|---|---|
| ber. | C 54,47 | H 6,03 | N 5,03 | S 7,67 | O 21,05 | Na 2,75 |
| gef. | C 54,26 | H 6,12 | N 5,00 | S 7,49 | | Na 2,48 |

Analog zur Synthese nach Beispiel 14-16 und 18-27 a (Festphasenpeptidsynthese) wird das VIP-Analogon HSDAVFTDNY TRLRKKMAVK KYLNSILN an der Festphase synthetisiert. Der Farbstoff 7-[5-N-(2,3-Dihydroxypropyl)aminocarbonyl-3,3-dimethyl-1-(4-sulfonatobutyl)indolin-2-yliden]-1,3,5-heptatrien-1-yl]-3,3-dimethyl-5-carboxy(3H)indolio]butansulfonat, Natriumsalz wird nach Beispiel 14-16 und 18-27 b (Farbstoffkopplung) N-terminal an das Peptid gekoppelt und nach Beispiel 14-16 und 18-27 c (Schutzgruppenabspaltung und Ablösung der Farbstoff-Peptid-Konjugate) wird dieses Konjugat isoliert und mittels HPLC gereinigt.

In analoger Weise können weitere unsymmetrische hydrophile Farbstoffe aus folgenden Bausteinen aufgebaut werden:

### Hydrophile Indolenin-Derivate mit Hydroxyalkylsubstituenten: (hergestellt nach Beispiel 44a)

a) 5-N-(2,3-Dihydroxypropyl)-N-methylaminocarbonyl-1-(4-sulfobutyl)-2,3,3-trimethyl(3H)indolenin
b) 5-N-(Hydroxyethyl)-aminocarbonyl-1-(4-sulfobutyl)-2,3,3-trimethyl(3H)indolenin
c) 5-N-(2,3-Dihydroxypropyl)-N-(hydroxyethyl)-aminocarbonyl-1-(4-sulfobutyl)-2,3,3-trimethyl(3H)indolenin
d) 5-N,N-(bis-Hydroxyethyl)-aminocarbonyl-1-(4-sulfobutyl)-2,3,3-trimethyl(3H)indolenin
e) 5-N-(2,3,4,5,6-Pentahydroxyhexyl)-aminocarbonyl-1-(4-sulfobutyl)-2,3,3-trimethyl(3H)indolenin
f) 5-N-(1,3,4-Trihydroxybut-2-yl)-N-methylaminocarbonyl-1-(4-sulfobutyl)-2,3,3-trimethyl(3H)indolenin

Indoleninderivate mit Carboxylgruppe:
a) 5-Carboxy-1-(4-sulfobutyl)-2,3,3-trimethyl(3H)indolenin
b) 5-Carboxymethyl-1-(4-sulfobutyl)-2,3,3-trimethyl(3H)indolenin
c) 5-Carboxy-1-(3-sulfopropyl)-2,3,3-trimethyl(3H)indolenin
d) 5-Carboxymethyl-1-(3-sulfopropyl)-2,3,3-trimethyl(3H)indolenin
e) 5-Carboxy-1-(2-sulfoethyl)-2,3,3-trimethyl(3H)indolenin
f) 5-Carboxymethyl-1-(2-sulfoethyl)-2,3,3-trimethyl(3H)indolenin

Dianilderivate zur Reaktion mit o. g. Indoleninen unter Bildung von Mono-, Di- oder Tricarbocyaninen:
a) Glutaconaldehyddianilid-hydrochlorid
b) Malonaldehyd-bis-phenylimin-hydrochlorid
c) N,N-Diphenylformamidin
d) N-[5-Anilino-2,4-(propan-1,3-diyl)-2,4-pentadien-1-yliden]anilinium-chlorid
e) N-[5-Anilino-2,4-(ethan-1,2-diyl)-2,4-pentadien-1-yliden]anilinium-chlorid
f) N-[5-Anilino-3-chlor-2,4-(propan-1,3-diyl)-2,4-pentadien-1-yliden]anilinium-chlorid
g) N-[5-Anilino-3-chlor-2,4-(ethan-1,2-diyl)-2,4-pentadien-1-yliden]anilinium-chlorid

### Beispiel 47

### 7-[5-N-(1,3,4-Trihydroxybut-2-yl)-aminocarbonyl-3,3-dimethyl-1-(4-sulfonatobutyl)indolin-2-yliden]- 3,5-(2-carboxypropan-1,3-diyl)-1,3,5-heptatrien-1-yl]-3,3-dimethyl-5- N-(1,3,4-trihydroxybut-2-yl)-aminocarbonyl(3H)indolio]butansulfonat, Natriumsalz, N-Hydroxysuccinimidester

### a) 4-[2-[4-Chlor-7-[5-N-(1,3,4-trihydroxybut-2-yl)aminocarbonyl-3,3-dimethyl-1-(4-sulfonatobutyl)indolin-2-yliden]-3,5-[2-(methoxycarbonyl)propan-1,3-diyl]-1,3,5-heptatrien-1-yl]-3,3-dimethyl-5-[N-(1,3,4-trihydroxybut-2-yl)aminocarbonyl] (3H)indolio]butansulfonat, Natriumsalz

Zu einer Lösung von 0,8 g (5 mmol) 4-(Methoxycarbonyl)-cyclohexanon in 5 ml Dichlormethan addiert man 5,5 ml (10 mmol) Phosphoroxychlorid und 6 ml N,N-Dimethylformamid bei 0°C. Anschließend erhitzt man eine Stunde am Rückfluß. Man destilliert das Dichlormethan ab und fügt bei maximal 5°C 4 ml Anilin in 10 ml Methanol hinzu. Man gießt die Reaktionsmischung auf Eis, gibt 5 ml konzentrierte Salzsäure hinzu und läßt das Zwischenprodukt fünf Stunden bei 0°C auskristallisieren. Man saugt die Kristalle ab und setzt diese ohne weitere Reinigung in die nächste Reaktion ein. Dazu löst man die Kristalle in wasserfreiem Ethanol und fügt 4,4 g (10 mmol) 5-N-(1,3,4-Trihydroxybut-2-yl)-N-methylaminocarbonyl-1-(4-sulfobutyl)-2,3,3-trimethyl(3H)indolenin (s. Beispiel 45) und 0,8 g wasserfreies Natriumacetat hinzu. Man erhitzt für eine Stunde am Rückfluß, filtriert den Feststoff ab und dampft das Filtrat zur Trockne ein. Der Rückstand wird zur Reinigung chromatographiert.
Ausbeute: 3,25 g (58 % d. Th.)

| Analyse (bezogen auf lösungsmittelfreie Substanz): | | | | | | | |
|---|---|---|---|---|---|---|---|
| ber. | C 54,90 | H 6,14 | Cl 3,18 | N 5,02 | S 5,75 | O 22,94 | Na 2,06 |
| gef. | C 54,76 | H 6,03 | Cl 2,99 | N 4,91 | S 5,60 | | Na 1,83 |

### b) 7-[5-N-(1,3,4-Trihydroxybut-2-yl)aminocarbonyl-3,3-dimethyl-1-(4-sulfonatobutyl)indolin-2-yliden]- 3,5-(2-carboxypropan-1,3-diyl)-1,3,5-heptatrien-1-yl]-3,3-dimethyl-5- N-(1,3,4-trihydroxybut-2-yl)aminocarbonyl(3H)indolio]butansulfonat, Natriumsalz

Eine Mischung von 10 mg (0,4 mmol) Natriumhydrid und 80 mg (1,3 mmol) Ethanthiol in 10 ml wasserfreiem N,N-Dimethylformamid werden unter Stickstoff 30 Minuten bei Raumtemperatur gerührt. Anschließend versetzt man mit 112 mg (0,1 mmol) 4-[2-[4-Chlor-7-[5-N-(2,3,4,5,6-pentahydroxyhexyl)aminocarbonyl-3,3-dimethyl-1-(4-sulfonatobutyl)indolin-2-yliden]-3,5-[2-(methoxycarbonyl)propan-1,3-diyl]-1,3,5-heptatrien-1-yl]-3,3-dimethyl5-[N-(2,3,4,5,6-pentahydroxyhexyl)-aminocarbonyl] (3H)indolio]butansulfonat, Natriumsalz (Beispiel 47a) in 3 ml N,N-Dimethylformamid. Die Reaktionsmischung wird für zwei Stunden auf 100°C erhitzt und nach Abkühlung auf Raumtemperatur mit Kohlendioxid gequencht. Man dampft am Rotationsverdampfer zur Trockne ein und extrahiert den Rückstand mit heißem Ethanol. Man dampft den Extrakt ein und chromatographiert das Rohprodukt.
Ausbeute: 75 mg (67 % d. Th.)

| Analyse (bezogen auf lösungsmittelfreie Substanz): | | | | | | |
|---|---|---|---|---|---|---|
| ber. | C 56,27 | H 6,33 | N 5,25 | S 6,01 | O 23,99 | Na 2,15 |
| gef. | C 56,13 | H 6,46 | N 5,12 | S 5,87 | | Na 1,88 |

### c) 7-[5-N-(1,3,4-Trihydroxybut-2-yl)-aminocarbonyl-3,3-dimethyl-1-(4-sulfonatobutyl)indolin-2-yliden]- 3,5-(2-carboxypropan-1,3-diyl)-1,3,5-heptatrien-1-yl]-3,3-dimethyl-5- N-(1,3,4-trihydroxybut-2-yl)-aminocarbonyl(3H)indolio]butansulfonat-N-Hydroxysuccinimidester, Natriumsalz

Zur Herstellung des Aktivesters löst man 14 mg (0,12 mmol) N-Hydroxysuccinimid und 64 mg (0,06 mmol) der Carbonsäure in 2 ml N,N-Dimethylformamid. Nach 15 Minuten setzt man 25 mg (0,12 mmol) Dicyclohexylcarbodiimid zu und rührt über Nacht bei Raumtemperatur. Der Aktivester wird mittels präparativer HPLC gereinigt.
Ausbeute: 60 mg (86 % d. Th.)

| Analyse (bezogen auf lösungsmittelfreie Substanz): | | | | | | |
|---|---|---|---|---|---|---|
| ber. | C 55,71 | H 6,06 | N 6,02 | S 5,51 | O 24,73 | Na 1,97 |
| gef. | C 55,59 | H 6,21 | N 5,93 | S 5,37 | | Na 1,75 |

Analog zur Synthese nach Beispiel 14-16 und 18-27 a (Festphasenpeptidsynthese) wird das VIP-Analogon HSDAVFTDNY TRLRKAMAVK KYLNSILN an der Festphase synthetisiert. Der Farbstoff 7-[5-N-(1,3,4-Trihydroxybut-2-yl)-aminocarbonyl-3,3-dimethyl-1-(4-sulfonatobutyl)indolin-2-yliden]- 3,5-(2-carboxypropan-1,3-diyl)-1,3,5-heptatrien-1-yl]-3,3-dimethyl-5-N-(1,3,4-trihydroxybut-2-yl)-aminocarbonyl(3H)indolio]butansulfonat, Natriumsalz wird nach Beispiel 14-16 und 18-27 b (Farbstoffkopplung) an das Peptid N-terminal gekoppelt und nach Beispiel 14-16 und 18-27 c (Schutzgruppenabspaltung und Ablösung der Farbstoff-Peptid-Konjugate) wird dieses Konjugat isoliert und gereinigt.

### Beispiel 48

### 7-[5-N-(1,3,4-Trihydroxybut-2-yl)-aminocarbonyl-3,3-dimethyl-1-(4-sulfonatobutyl)indolin-2-yliden]- 3,5-(2-carboxypropan-1,3-diyl)-1,3,5-heptatrien-1-yl]-3,3-dimethyl-5- N-(1,3,4-trihydroxybut-2-yl)-aminocarbonyl(3H)indolio]butansulfonat, Natriumsalz

### a) 5-N-(11-Aminoundecyl)aminocarbonyl-1-(4-sulfobutyl)-2,3,3-trimethyl(3H)indolenin

340 mg (1 mmol) 5-Carboxy-1-(4-sulfobutyl)-2,3,3-trimethyl(3H)indolenin (Anal. Biochem. 217,197, 1994) werden in 5 ml absolutem N,N-Dimethylformamid und 1 ml Pyridin vorgelegt und mit 0,5 g (2 mmol) Disuccinimidylcarbonat versetzt. Nach drei Stunden addiert man 0,805 g (4 mmol) 11-Aminoundecansäure. Man rührt über Nacht bei Raumtemperatur, dampft den Ansatz zur Trockne ein und rührt den Rückstand mit Diethylether aus. Der Feststoff wird abgesaugt und zur Reinigung an RP-Material chromatographiert.
Ausbeute: 0,37 g (71 % d. Th.)

| Analyse (bezogen auf lösungsmittelfreie Substanz): | | | | | |
|---|---|---|---|---|---|
| ber. | C 62,04 | H 8,10 | N 5,36 | S 6,13 | O 18,37 |
| gef. | C 61,88 | H 8,23 | N 5,17 | S 6,02 | |

### b) 7-[5-N-(1,3,4-Trihydroxybut-2-yl)-aminocarbonyl-3,3-dimethyl-1-(4-sulfonatobutyl)indolin-2-yliden]- 3,5-(2-carboxypropan-1,3-diyl)-1,3,5-heptatrien-1-yl]-3,3-dimethyl-5-N-(1,3,4-trihydroxybut-2-yl)-aminocarbonyl(3H)indolio]butansulfonat, Natriumsalz

Eine Lösung von 0,35 g (0,67 mmol) 5-N-(11-Aminoundecyl)aminocarbonyl-1-(4-sulfobutyl)-2,3,3-trimethyl(3H)indolenin (Beispiel 48a) und 0,18 g (0,645 mmol) Glutaconaldehyddianil in 3 ml Essigsäureanhydrid werden 20 Minuten bei 110°C gerührt. Anschließend addiert man 344 mg (0,83 mmol) 5-N-(2,3-Dihydroxypropyl)-aminocarbonyl-1-(4-sulfobutyl)-2,3,3-trimethyl(3H-)indolenin (Beispiel 44a), 0,2 g Natriumacetat, 3 ml Essigsäureanhydrid und 1 ml Essigsäure. Man läßt die Reaktionsmischung zwei Stunden bei 110°C rühren, kühlt auf Raumtemperatur ab und fällt das Produkt mit Diethylether. Das Rohprodukt wird über RP-Material chromatographiert. Ausbeute: 0,41 g (60 % d. Th.)

| Analyse (bezogen auf lösungsmittelfreie Substanz): | | | | | | |
|---|---|---|---|---|---|---|
| ber. | C 60,10 | H 7,02 | N 5,50 | S 6,29 | O 18,84 | Na 2,26 |
| gef. | C 59,96 | H 7,14 | N 5,33 | S 6,15 | | Na 2,11 |

Analog zur Synthese nach Beispiel 14-16 und 18-27 a (Festphasenpeptidsynthese) wird das VIP-Analogon HSDAVFTDNY TRLRKQMWVK KYLNSILN an der Festphase synthetisiert. Der Farbstoff 7-[5-N-(1,3,4-Trihydroxybut-2-yl)-aminocarbonyl-3,3-dimethyl-1-(4-sulfonatobutyl)indolin-2-yliden]- 3,5-(2-carboxypropan-2,3-diyl)-1,3,5-heptatrien-1-yl]-3,3-dimethyl-5- N-(1,3,4-trihydroxybut-2-yl)-aminocarbonyl(3H)indolio]butansulfonat, Natriumsalz wird nach Beispiel 14-16 und 18-27 b (Farbstoffkopplung) an das Peptid N-terminal gekoppelt und nach Beispiel 14-16 und 18-27 c (Schutzgruppenabspaltung und Ablösung der Farbstoff-Peptid-Konjugate) wird dieses Konjugat isoliert und gereinigt.

Weitere erfindungsgemäße Farbstoffe können nach Beispiel 44a und b hergestellt werden, in dem man an Stelle von 11-Aminoundecansäure in Beispiel 48a folgende Aminosäuren und die in Beispiel 46 genannten Indoleninderivate mit Hydroxyalkylsubstituenten a)-f) verwendet:
i. Glycin
ii. Alanin
iii. β-Alanin
iv. 4-Aminobutansäure
v. 6-Aminohexansäure
vi. H₂N-(CH₂CH₂O)₃CH₂COOH (TH 53, 20, 6977)
vii. H₂N-(CH₂CH₂O)₄CH₂COOH (JOC 63, 5, 1728, 1998)
viii. H₂N-CH₂CH₂COO(CH₂CH₂O)₄-CO-CH₂CH₂COOH (Lett. Pept. Sci. 6, 135, 1999)
ix. HCl* H₂N-PEG-COOH (MW 3400 g/mol; Shearwater Polymers Inc., USA)

### Beispiel 49

### 4-[2-[4-(4-(N-(4-Aza-6-brom-5-oxohexyl)aminocarbonylethyl)phenyloxy)-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)aminocarbonyl-3,3-dimethyl(3H)indolio]butansulfonat, Natriumsalz

### a) [3-[N-(tert.-Butoxycarbonyl)amino]propyl]-N'-(bromacetyl)-amid

2,5 g (14,4 mmol) [3-[N-(tert.-Butoxycarbonyl)amino]-propyl]amin werden in 15 ml Dioxan gelöst und nach Zugabe von 4,4 ml Triethylamin bei 0°C mit 3,2 g (16 mmol) Bromacetylbromid versetzt. Man rührt über Nacht bei Raumtemperatur und addiert anschließend weitere 320 mg Bromacetylbromid. Nach zwei Stunden bei Raumtemperatur wird der Niederschlag abgesaugt, die Lösung eingeengt und der Rückstand in Essigsäureethylester aufgenommen. Man wäscht mit Wasser und trocknet die organische Phase über Natriumsulfat.
Ausbeute: 3,2 g (75 % d. Th.)

| Analyse (bezogen auf lösungsmittelfreie Substanz): | | | | | |
|---|---|---|---|---|---|
| ber. | C 40,69 | H 6,49 | Br 27,07 | N 9,49 | O 16,26 |
| gef. | C 40,50 | H 6,37 | Br 26,89 | N 9,58 | |

### b) [3-[N-( Bromacetyl)amino)propyl]amin, Hydrochlorid

3,1 g (10,5 mmol) [3-[N-(tert.-Butoxycarbonyl)amino- ]propyl]-N'-(bromacetyl)-amid (Beispiel 49a) werden mit 50 mmol 1M Salzsäure in Essigsäureethylester für fünf Stunden bei Raumtemperatur gerührt. Man saugt das Produkt ab und wäscht den Feststoff mit Essigsäureethylester nach.
Ausbeute: 2,3 g (95 % d. Th.)

| Analyse (bezogen auf lösungsmittelfreie Substanz): | | | | | | |
|---|---|---|---|---|---|---|
| ber. | C 25,94 | H 5,22 | Cl 15,31 | Br 34,51 | N 12,10 | O 6,91 |
| gef. | C 25,76 | H 5,41 | Cl 15,55 | Br 34,34 | N 11,97 | |

### c) 4-[2-[4-(4-(N-(4-Aza-6-brom-5-oxo-hexyl)aminocarbonylethyl)phenyloxy)-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)aminocarbonyl-3,3-dimethyl(3H)indolio]butansulfonat, Natriumsalz

121 mg (0,1 mmol) 4-[2-[4-(4-(2-Carboxyethyl)phenyloxy)-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)-aminocarbonyl-3,3-dimethyl(3H)indolio]butansulfonat, Natriumsalz, N-Hydroxysuccinimidester (Beispiel 44c) werden in 0,5 ml N,N-Dimethylformamid gelöst, mit 0,06 ml Triethylamin und 70 mg (0,3 mmol) [3-[N-( Bromacetyl)amino]propyl]amin, Hydrochlorid '(Beispiel 49b) versetzt.
Man rührt vier Stunden bei 60°C, kühlt anschließend auf Raumtemperatur ab und fällt das Produkt mit Diethylether. Der Feststoff wird abgesaugt und mit reichlich Diethylether gewaschen.
Ausbeute: 0,11 mg (80 % d. Th.)

| Analyse (bezogen auf lösungsmittelfreie Substanz): | | | | | | | |
|---|---|---|---|---|---|---|---|
| ber. | C 56,17 | H 6,18 | Br 6,13 | N 6,44 | S 4,92 | Na 1,76 | O 18,40 |
| gef. | C 55,96 | H 6,26 | Br 6,01 | N 6,27 | S 4,81 | Na 1,53 | |

Analog zur Synthese nach Beispiel 14-16 und 18-27 a (Festphasenpeptidsynthese) wird das VIP-Analogon HSDAVFTDNY TRLRKQCAVK KYLNSLLN an der Festphase synthetisiert. Das Farbstoffbromid 4-[2-[4-(4-(N-(4-Aza-6-brom-5-oxo-hexyl)aminocarbonylethyl)phenyloxy)-7-[5-N-(dihydroxypropyl)aminocarbonyl-3,3-dimethyl-1-(4-sulfonatobutyl)indolin-2-yliden]-3,5-(propan-1,3-diyl)-1,3,5-heptatrien-1-yl]-5-N-(dihydroxypropyl)aminocarbonyl-3,3-dimethyl(3H)indolio]butansulfonat, Natriumsalz wird nach Beispiel 17 an das Peptid N-terminal gekoppelt und nach Beispiel 14-16 und 18-27 c (Schutzgruppenabspaltung und Ablösung der Farbstoff-Peptid-Konjugate) wird dieses Konjugat isoliert und gereinigt.

## Patentansprüche

1. Cyaninfarbstoffe der allgemeinen Formel XVIII, 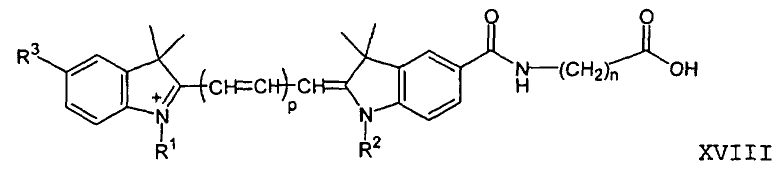 worin
p für 1, 2 oder 3,
n für 1, 2, 3, 4 oder 10 steht,
R¹ und R² unabhängig voneinander für einen 4-Sulfobutyl-, 3-Sulfopropyl-, 2-Sulfoethyl-, 3-Methyl-3-sulfopropyl-, Methyl-, Ethyl- oder Propylrest stehen, und
R³ für Wasserstoff oder für einen Rest -COOE¹, -CONE¹E², -NHCOE¹, -NHCONHE¹, -NE¹E², -OE¹, -OSO₃E¹, -SO₃E¹, -SO₂NHE¹,
wobei E¹ und E² unabhängig voneinander für ein Wasserstoffatom oder für einen Methyl-, Ethyl- oder einen C₃-C₆-Alkylrest, der von 0 bis 2 Sauerstoffatomen und/oder von 0 bis 1 Carbonylgruppen unterbrochen ist und/oder mit 0 bis 5 Hydroxygruppen substituiert ist, stehen,
steht.

2. Cyaninfarbstoffe der allgemeinen Formel XIX oder XX 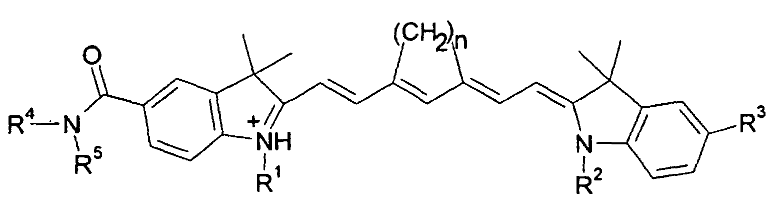 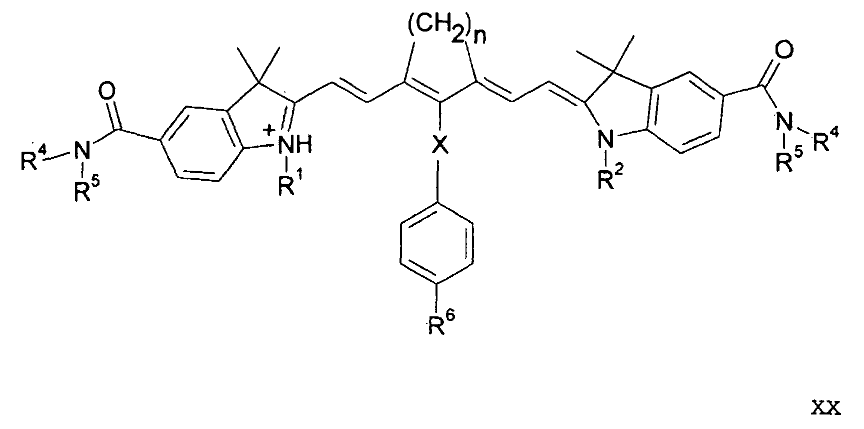 worin
n für 2 oder 3 steht,
R¹ und R² unabhängig voneinander einen 4-Sulfobutyl-, 3-Sulfopropyl- oder 2-Sulfoethylrest darstellen,
R³ für eine -COOH-Gruppe oder einen der folgenden Reste steht:
-CONH-(CH₂)ₙ-COOH mit n = 2 oder 3,
-CONH-(CH₂)ₙ-NCS mit n = 2 oder 3,
-CONH-(CH₂)ₙ-NHCO-CH₂-X¹ mit n = 2 oder 3 und X¹ = Cl, Br, I 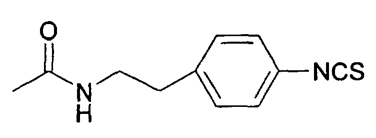
R⁴ und R⁵ unabhängig voneinander für ein Wasserstoffatom, einen Methylrest oder einen hydroxylierten Alkylrest wie z.B. 1-Hydroxyethyl, 1-Hydroxypropyl, 2,3-Dihydroxypropyl, 1,3-Dihydroxy-2-propyl, 1,2,3-Trihydroxybutyl, 1,3,4-Trihydroxy-2-butyl, 1,2,3,4,5-Pentahydroxyhexyl stehen,
R⁶ für eine der folgenden Gruppen steht:
-(CH₂)ₘ-COOH mit m = 0 bis 2,
-(CH₂)ₘ-NCS mit m = 0 bis 2,
-(CH₂)ₘ-CONH-Peptid mit m = 0 bis 2,
-(CH₂)ₘ-NH-CS-NH-Peptid mit m = 0 bis 2,
und X für ein Sauerstoffatom oder ein Schwefelatom steht.

3. Cyaninfarbstoffe der allgemeinen Formel XXI 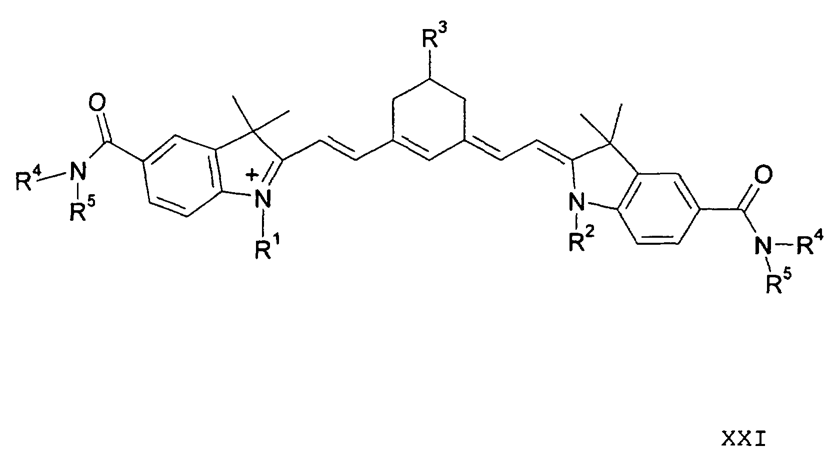 worin
R¹ und R² unabhängig voneinander für einen 4-Sulfobutyloder 3-Sulfopropylrest darstellen,
R³ für eine -COOH-Gruppe oder einen der folgenden Reste steht:
-CONH-(CH₂)ₙ-COOH mit n = 2 oder 3,
-CONH-(CH₂)ₙ-NCS mit n = 2 oder 3,
-CONH-(CH₂)ₙ-NHCO-CH₂-X¹ mit n = 2 oder 3 und X¹ = Cl, Br, I 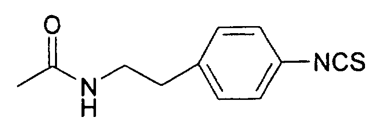
und R⁴ und R⁵ unabhängig voneinander für ein Wasserstoffatom, einen Methylrest oder einen hydroxylierten Alkylrest wie z.B. 1-Hydroxyethyl, 1-Hydroxypropyl, 2,3-Dihydroxypropyl, 1,3-Dihydroxy-2-propyl, 1,2,3-Trihydroxybutyl, 1,3,4-Trihydroxy-2-butyl, 1,2,3,4,5-Pentahydroxyhexyl stehen.

## Claims

1. Cyanine dyes of the general formula XVIII, 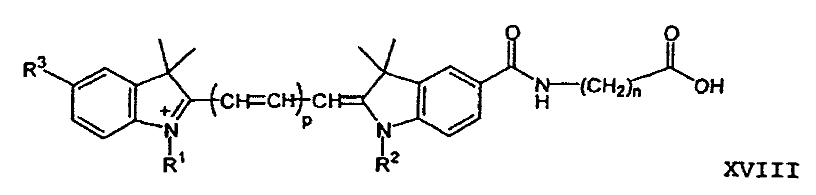 in which
p is 1, 2 or 3,
n is 1, 2, 3, 4 or 10,
R¹ and R² are, independently of one another, a 4-sulphobutyl, 3-sulphopropyl, 2-sulphoethyl, 3-methyl-3-sulphopropyl, methyl, ethyl or propyl radical, and
R³ is hydrogen or is a radical -COOE¹, -CONE¹E², -NHCOE¹, -NHCONHE¹, -NE¹E², -OE¹, -OSO₃E¹, -SO₃E¹, -SO₂NHE¹,
where E¹ and E² are, independently of one another, a hydrogen atom or a methyl, ethyl or a C₃-C₆-alkyl radical which is interrupted by from 0 to 2 oxygen atoms and/or from 0 to 1 carbonyl groups, and/or is substituted by 0 to 5 hydroxyl groups.

2. Cynanine dyes of the general formula XIX or XX 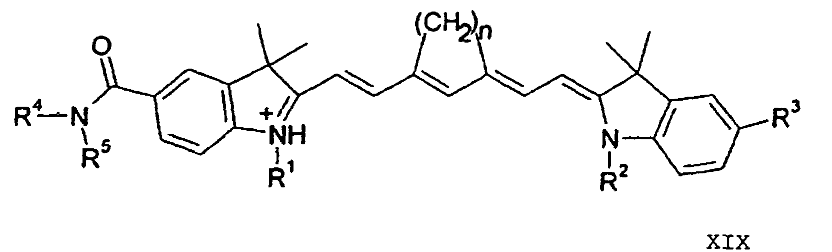 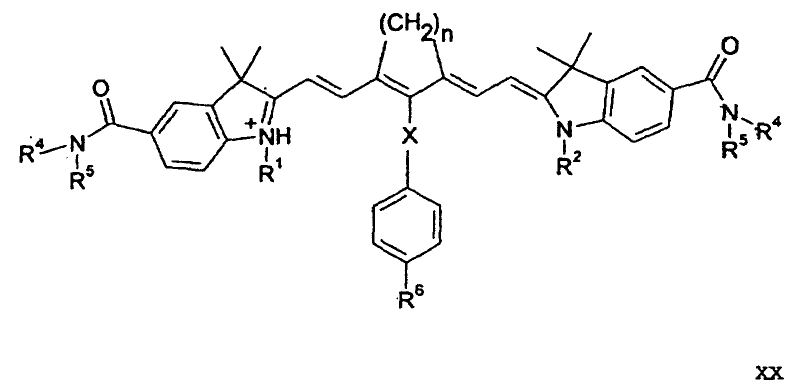 in which
n is 2 or 3,
R¹ and R² are, independently of one another, a 4-sulphobutyl, 3-sulphopropyl or 2-sulphoethyl radical,
R³ is a -COOH group or one of the following radicals:
-CONH-(CH₂)ₙ-COOH with n = 2 or 3,
-CONH-(CH₂)ₙ-NCS with n = 2 or 3,
-CONH-(CH₂)ₙ-NHCO-CH₂-X¹ with n = 2 or 3 and X¹ = Cl, Br, I 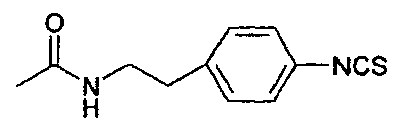
R⁴ and R⁵ are, independently of one another, a hydrogen atom, a methyl radical or a hydroxylated alkyl radical such as, for example, 1-hydroxyethyl, 1-hydroxypropyl, 2,3-dihydroxypropyl, 1,3-dihydroxy-2-propyl, 1,2,3-trihydroxybutyl, 1,3,4-trihydroxy-2-butyl, 1,2,3,4,5,-pentahydroxyhexyl,
R⁶ is one of the following groups:
-(CH₂)ₘ-COOH with m = 0 to 2,
-(CH₂)ₘ-NCS with m = 0 to 2,
-(CH₂)ₘ-CONH-peptide with m = 0 to 2,
-(CH₂)ₘ-NH-CS-NH-peptide with m = 0 to 2,
and X is an oxygen atom or a sulphur atom.

3. Cyanine dyes of the general formula XXI 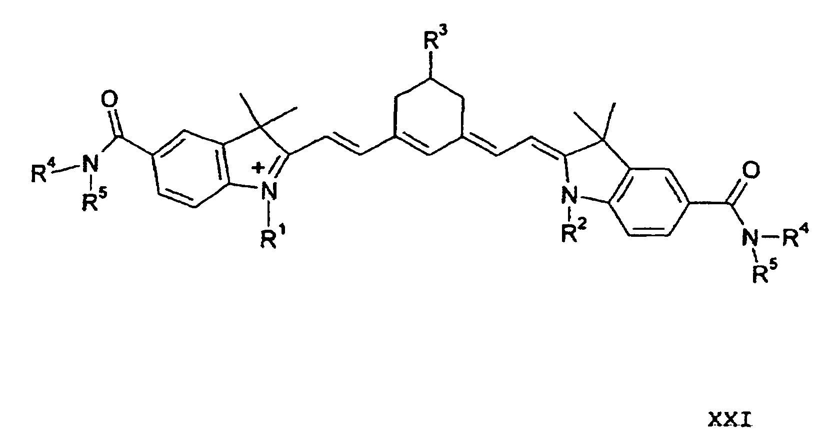 in which
R¹ and R² are, independently of one another, a 4-sulphobutyl or 3-sulphopropyl radical,
R³ is a -COOH group or one of the following radicals:
-CONH-(CH₂)ₙ-COOH with n = 2 or 3,
-CONH-(CH₂)ₙ-NCS with n = 2 or 3,
-CONH-(CH₂)ₙ-NCHO-CH₂-X¹ with n = 2 or 3 and X¹ = Cl, Br, I 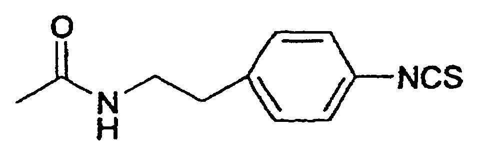
and R⁴ and R⁵ are, independently of one another, a hydrogen atom, a methyl radical or a hydroxylated alkyl radical such as, for example, 1-hydroxyethyl, 1-hydroxypropyl, 2,3-dihydroxypropyl, 1,3-dihydroxy-2-propyl, 1,2,3-trihydroxybutyl, 1,3,4-trihydroxy-2-butyl, 1,2,3,4,5-pentahydroxyhexyl.

## Revendications

1. Colorants cyanine de formule générale XVIII, 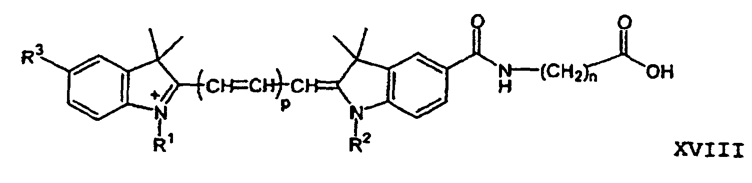 dans laquelle
p vaut 1, 2 ou 3,
n vaut 1, 2, 3, 4 ou 10,
R¹ et R² représentent, indépendamment l'un de l'autre, un radical 4-sulfobutyle, 3-sulfopropyle, 2-sulfoéthyle, 3-méthyl-3-sulfopropyle, méthyle, éthyle ou propyle, et R³ représente un hydrogène ou un radical -COOE¹, -CONE¹E², -NHCOE¹, -NHCONHE¹, -NE¹E², -OE¹, -OSO₃E¹, -SO₃E¹, -SO₂NHE¹,
où E¹ et E² représentent, indépendamment l'un de l'autre, un atome d'hydrogène ou un radical méthyle, éthyle ou alkyle en C₃-C₆, qui est interrompu par de 0 à 2 atomes d'oxygène et/ou de 0 à 1 groupe carbonyle et/ou substitué par de 0 à 5 groupes hydroxyde.

2. Colorants cyanine de formules générales XIX ou XX 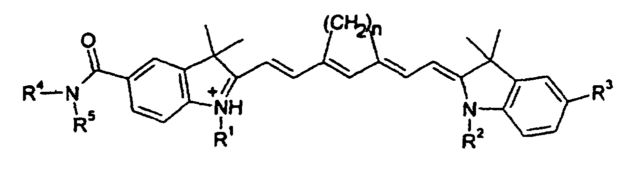 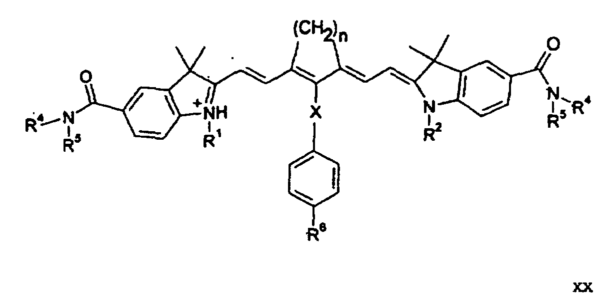 dans lesquelles
n vaut 2 ou 3,
R¹ et R² représentent, indépendamment l'un de l'autre, un radical 4-sulfobutyle, 3-sulfopropyle ou 2-sulfoéthyle,
R³ représente un groupe -COOH ou l'un des radicaux suivants :
-CONH-(CH₂)ₙ-COOH avec n = 2 ou 3,
-CONH-(CH₂)ₙ-NCS avec n = 2 ou 3,
-CONH-(CH₂)ₙ-NHCO-CH₂-X¹ avec n = 2 ou 3 et X¹ = Cl, Br, I
R⁴ et R⁵ représentent, indépendamment l'un de l'autre, un atome d'hydrogène, un radical méthyle ou un radical alkyle hydroxylé tel que, par exemple, 1-hydroxyéthyle, 1-hydroxypropyle, 2,3-dihydroxypropyle, 1,3-dihydroxy-2-propyle, 1,2,3-trihydroxybutyle, 1,3,4-trihydroxy-2-butyle, 1,2,3,4,5-pentahydroxyhexyle,
R⁶ représente l'un des groupes suivants :
-(CH₂)ₘ-COOH avec m = 0 à 2,
-(CH₂)ₘ-NCS avec m = 0 à 2,
-(CH₂)ₘ-CONH-peptide avec m = 0 à 2,
-(CH₂)ₘ-NH-CS-NH-peptide avec m = 0 à 2,
et X représente un atome d'oxygène ou un atome de soufre.

3. Colorants cyanine de formule générale XXI 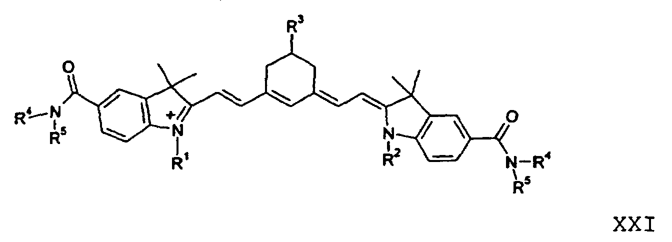 dans laquelle
R¹ et R² représentent, indépendamment l'un de l'autre, un radical 4-sulfobutyle ou 3-sulfopropyle,
R³ représente un groupe -COOH ou l'un des radicaux suivants :
-CONH-(CH₂)ₙ-COOH avec n = 2 ou 3,
-CONH-(CH₂)ₙ-NCS avec n = 2 ou 3,
-CONH-(CH₂)ₙ-NHCO-CH₂-X¹ avec n = 2 ou 3 et X¹ = Cl, Br, I 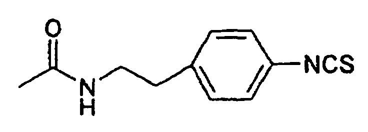
et R⁴ et R⁵ représentent, indépendamment l'un de l'autre, un atome d'hydrogène, un radical méthyle ou un radical alkyle hydroxylé tel que, par exemple, 1-hydroxyéthyle, 1-hydroxypropyle, 2,3-dihydroxypropyle, 1,3-dihydroxy-2-propyle, 1,2,3-trihydroxybutyle, 1,3,4-trihydroxy-2-butyle, 1,2,3,4,5-pentahydroxyhexyle.
